# EUROPEAN PATENT SPECIFICATION

(11) **EP 4 408 391 B1**
(45) Date of publication and mention of the grant of the patent: **04.11.2026**
(21) Application number: 22797712.1
(22) Date of filing: 28.09.2022
(51) Int. Cl.: A61K 9/00, A61K 9/06, A61K 31/52, A61K 47/10, A61K 47/36, A61K 47/38, A61P 17/02

(54) **TOPICAL COMPOSITIONS OF 2-PHENYL-3,4-DIHYDROPYRROLO[2,L-F] [1,2,4]TRIAZINONE DERIVATIVES AND USES THEREOF**
TOPISCHE ZUSAMMENSETZUNGEN VON 2-PHENYL-3,4-DIHYDROPYRROLO[2,L-F]-[1,2,4]TRIAZINONDERIVATE UND IHRE VERWENDUNG
COMPOSITIONS TOPIQUES DE DÉRIVÉS DE 2-PHÉNYL-3,4-DIHYDROPYRROLO[2,L-F][1,2,4]TRIAZINONE ET LEURS UTILISATIONS

(30) Priority: 29.09.2021 EP 21199822
(43) Date of publication of application: 07.08.2024
(73) Proprietor: Topadur Pharma AG, 8952 Schlieren (CH)
(72) Inventor: TENOR, Hermann, 8952 Schlieren (CH); LUDIN, Christian, 8952 Schlieren (CH); BOUVET, Raphael, 8952 Schlieren (CH); CRACOWSKI, Jean Luc, 8952 Schlieren (CH)
(74) Representative: Latscha Schöllhorn Partner AG
(86) International application number: PCT/EP2022/076952
(87) International publication number: WO 2023/052407

(56) References cited:
- WO-A1-2017/085056
- VALENTA C ET AL: "The use of polymers for dermal and transdermal delivery", EUROPEAN JOURNAL OF PHARMACEUTICS AND BIOPHARMACEUTICS, ELSEVIER SCIENCE PUBLISHERS B.V., AMSTERDAM, NL, vol. 58, no. 2, 1 September 2004 (2004-09-01), pages 279 - 289, XP004526312, ISSN: 0939-6411, DOI: 10.1016/J.EJPB.2004.02.017
- PEARSON DAVID R ET AL: "Systemic sclerosis: Current concepts of skin and systemic manifestations", CLINICS IN DERMATOLOGY, J.B. LIPPINCOTT, PHILADELPHIA, PA, US, vol. 36, no. 4, 12 April 2018 (2018-04-12), pages 459 - 474, XP085416674, ISSN: 0738-081X, DOI: 10.1016/J.CLINDERMATOL.2018.04.004

## Description

The present invention relates to topical compositions comprising compounds of formula I and thus 2-phenyl-3,4-dihydropyrrolo[2,1-f][1,2,4]triazinone derivatives. The invention also relates to said topical compositions for use in the treatment of diseases or disorders alleviated by inhibition of PDE5 and/or NO related endothelial dysfunction in a subject, preferably in a human, and particularly, for the treatment of vascular diseases and disorders, in particular peripheral vascular diseases and disorders including for wound and chronic wound healing.

### RELATED ART

The following discussion of the related art of the disclosure is merely provided to aid the reader in understanding the disclosure and is not admitted to describe or constitute related and art to the present disclosure.

Phosphodiesterases (PDEs) are enzymes that catalyzes the hydrolysis and thus the degradation of cyclic adenosine monophosphate (cAMP) and cyclic guanosine monophosphate (cGMP) and thereby regulates intracellular levels of second messengers. Inhibition of PDEs leads to increasing intracellular concentrations of endogenous cAMP/cGMP. Therefore, inhibition of PDE can mediate a variety of physiological mechanisms at different cell and organ levels. Phosphodiesterase type 5 (PDE5) hydrolyses cyclic guanylate monophosphate (cGMP) specifically to 5' GMP. The selective inhibition of PDE5 has been validated as a relevant approach and strategies directed to promote inhibition of PDE5 activity have been applied as therapeutic tools, in particular, in neuronal, vascular and cardiovascular diseases and disorders. Moreover, the introduction of PDE5 inhibitors has revolutionized the treatment of male erectile dysfunction (Andersson KE, British Journal of Pharmacology (2018) 175:2554-2565; Das A et al. Pharmacol Ther. (2015) 147:12-21; Dobhal T et al. Critical Review in Pharmaceutical Sciences (2012) 1(3):13-27). Most prominent examples of PDE5 inhibitors are Sildenafil, Tadalafil and Vardenafil, which have been described among others, for example, in WO 99/24433, WO 01/60825, EP 995'751 and WO 2011/075655. Recently a novel class of PDE 5 inhibitors with dual-pharmacology releasing NO in addition to its PDE 5 inhibition in a more than additive fashion has been described (WO 2017/085056).

Systemic sclerosis (SSc, ICD-10 M34) is a rare, complex, multi organ, autoimmune, chronic connective tissue disease of unknown etiology characterized by vascular abnormalities and diffuse fibrosis in the skin, joints, and internal organs (especially the oesophagus with gastroesophageal reflux disease (GERD) and dysphagia, lower gastrointestinal tract, lungs, heart, and kidneys). Common symptoms include (secondary) Raynaud's phenomenon (RP), digital ulcers (DU), polyarthralgia, dysphagia, heartburn, and swelling and eventually skin tightening and contractures of the fingers. Lung, heart, and kidney involvement accounts for most deaths (Allanore Y et al. Nat Rev Dis Primers (2015) 1:15002; Pearson DR et al. Clin Dermatol (2018) 36(4):459-474; Denton CP and Khanna D, Lancet (2017) 390:1685-1699). Digital ulcers (DU) that are often located at the fingertips or extensor areas of proximal interphalangeal joints are a frequent clinical manifestation in SSc. Digital ulcers are defined as a loss of epidermal covering with a break in the basement membrane (which separates dermis from epidermis). Clinically digital ulcers in SSc appear as a wound with visible blood vessels, fibrin, granulation tissue and/or underlying deeper structures (e.g., muscle, ligament, fat) or as it would appear on debridement. Digital ulcers substantially contribute to the disease burden in systemic sclerosis also because in general, they are painful and disabling, limiting HRQoL (Amanzi L et al. Rheumatology (Oxford) (2010) 49(7):1374-1382; Li W and Frech TM, J Scleroderma Relat Disord. (2017) 2(2):69-71; Hughes M et al. Nat Rev Rheumatol. (2020) 16(4):208-221). DU derived from digital pitting scars (DPS) are hidden below hyperkeratotic layers of DPS. Digital pitting scars are defined as small hyperkeratotic lesions overlying a cutaneous depression resulting from chronic ischemia. Such ulcers are rather small, superficial (not full thickness wounds) with predilections sites at fingertips and dorsal regions of digits. In the perilesional skin there are signs of inflammation and oedema. DU derived from DPS are associated with spontaneous pain. Such superficial DUs have a mean time to healing of less than one month and in general complications as gangrene or auto-amputation do not occur. Ischemia is assumed to account in part for DU derived from DPS.

Cutaneous signs and symptoms of SSc are of particular importance as they are the earliest, most frequent and most characteristic manifestations and recognized before systemic manifestations, thus allowing earlier initiation of management and treatment (Pearson DR et al. Clin Dermatol (2018) 36(4):459-474; Herrick et al. Exp Dermatol.(2020) 29:1144-1153). As a general approach, patients should be managed using non-pharmacological interventions such as patient education as first-line treatment, followed by pharmacological and surgical interventions. Regarding pharmacological interventions, systemic vasoactive therapies attempt to address the underlying factors that are implicated in the pathogenesis of SSc such as DU and RP are often used as a first-line therapy, although oral phosphodiesterase type 5 (PDE5) inhibitors such as sildenafil are increasingly used early on in the treatment of SSc and SSc-associated digital vasculopathy. However, vasodilatory adverse effects are not uncommon with systemic vasoactive therapies and PDE5 inhibitors. For example, oral sildenafil display common adverse drug reactions such as vital signs, namely systemic arterial blood pressure, heart rate and ECG, let alone that these adverse drug reactions are more common in patients receiving higher possible doses than lower possible doses (Hughes M et al. Nat Rev Rheumatol. (2020) 16(4):208-22). Moreover, a recent placebo-controlled trial of the PDE5 inhibitor sildenafil observed no difference in ulcer healing between placebo and sildenafil (Hachullan E. et al. Ann. Rheum. Dis. (2016) 75, 1009-1015).

Therapies to topically treat SSc Digital Ulcer or Reynolds Phenomena were explored in experimental clinical trials using glyceryl trinitrate in concentrations between 0.9% up to 2% (Hughes M et al. Microvascular Research (2017) 111:32-36; Hummers LK, et al. Ann Rheum Dis (2013) 72:1962-1967). Excipients in the used water-based formulations were lanolin- or 50% lecithin. The topical formulations were described as unpleasant. Moreover, described treatment side effects of glyceryl trinitrate were headiness, dizziness, vasodilatory side effect and pain. Other experimental clinicals trial for topical treatment of digital ulcers were described using very high concentrations of a formulation of D-α-tocopheryl acetate (96.0%) and micronized silica (4.0%) as gel, or using a topically applied dimethyl sulfoxide solution. However, none of these topical applications are approved for treatment of DU by a Regulatory Authority or are recommended by EULAR recommendations for the treatment of systemic sclerosis (Fiori G et al. Clinical and Experimental Rheumatology (2009) 27(Suppl 54):S51-S54; Williams DE et al. Arthritis Rheum. (1985) 28(3):308-14; Kowal-Bielecka O et al, Ann Rheum Dis. (2017) 76(8):1327-1339).

Thus, there is still a strong and unmet medical need for treatment of vascular diseases and disorders, and in particular peripheral vascular diseases and disorders including for wound and chronic wound healing.

### SUMMARY OF THE INVENTION

The inventors have surprisingly found and shown that topically, on wound treatment of the compounds and compositions of the invention demonstrated efficacy and improved wound healing in uPAR deficient mice, a disease-relevant animal model of systemic sclerosis having cutaneous ulcers. Further surprisingly, the inventors were able to provide inventive topical compositions according to the claims, in particular inventive topical compositions comprising Compound **1,** despite the shown de-facto non-solubility of the inventive compounds of formula **I,** in particular of Compound **1** in physiological aqueous phosphate buffer (pH 7.5). Moreover, the inventive topical compositions showed efficacy and improved wound healing in the disease-relevant animal model of systemic sclerosis having cutaneous ulcers at surprisingly very low concentrations of the inventive compounds of formula **I,** in particular of Compound **1.**

Moreover, it has been proven that local and topical administration rather than systemic exposure of the inventive compounds caused the improved wound healing. This is particularly advantageous since systemic or oral delivery of the prior art drugs has its limitations not only due to vascular abnormalities and diffuse fibrosis in the skin, but also due to adverse drug reactions observed with the systemic or oral vasodilators. Therefore, suboptimal pharmacokinetics and/or pharmacodynamics associated with systemic drug exposure such as systemic arterial hypotension are avoided or very unlikely to occur by and with the inventive topical administration of compounds and compositions of the invention. Moreover, the topical administration directly to the primary site of action allows application of efficacious therapeutic doses of the inventive compounds to be significantly lower as compared to systemic or oral administration, and thus to integrate local efficacy on wound with minimal systemic exposure. Finally, the topical delivery (at home) is a more convenient mode of administration than intravenous administration or an oral administration (medical ward).

Thus, in a first aspect, the present invention provides a topical composition comprising a compound of formula **I,** preferably an effective amount of a compound of formula **I,** or a pharmaceutically acceptable salt thereof 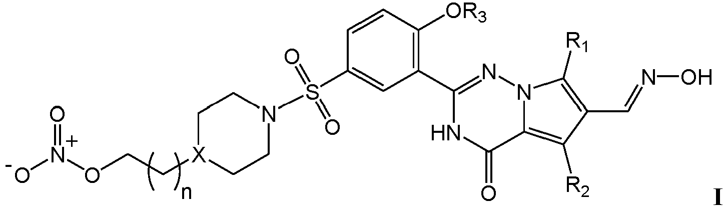 wherein
R₁ is ethyl or propyl, preferably propyl;
R₂ is methyl or ethyl, preferably methyl;
R₃ is ethyl or propyl, preferably propyl;
X is N or CH, preferably CH;
n is 1 or 2, preferably n=1;
wherein said topical composition comprises a solvent; and wherein said solvent comprises PEG 400, and wherein the amount of said PEG 400 is from about 60% to about 75% (w/w) based on the total weight of the topical composition, wherein the term about means ±10%.

In another aspect, the present invention provides a topical composition preferably a topical composition for on wound administration, comprising
(a) a compound of formula **I,** preferably an effective amount of a compound of formula **I,** or a pharmaceutically acceptable salt thereof 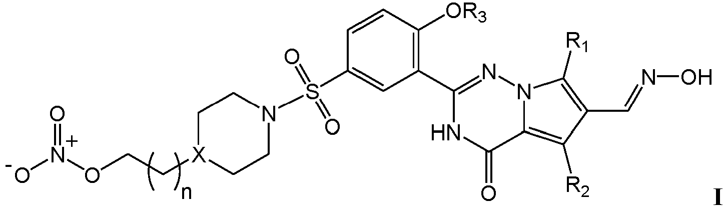 wherein
   R₁ is ethyl or propyl, preferably propyl;
   R₂ is methyl or ethyl, preferably methyl;
   R₃ is ethyl or propyl, preferably propyl;
   X is N or CH, preferably CH;
   n is 1 or 2, preferably n=1;
(b) a solvent; and
(c) optionally one or more other pharmaceutically acceptable excipients.

The topical composition of the present invention can be used for the treatment of diseases or disorders of the skin being mediated by or resulting from PDE5 activity and/or NO related endothelial dysfunction, in particular, such as ischemic skin ulcers, such as digital ulcers (DU) in systemic sclerosis, diabetic foot ulcer, leg ulcer, ischemic arterial ulcers, livedoid vasculopathy, Martorell hypertensive ischemic leg ulcer, thromboangiititis obliteragans (Buerger's disease), sickle cell leg ulcer, all of the foregoing preferably for wound healing, further preferably for chronic wound healing.

Thus, in a further aspect, the present invention provides the topical composition of the present invention for use in a method of topically treating a disease or disorder mediated by PDE5 activity and/or NO related endothelial dysfunction, preferably of a disease or disorder of the skin mediated by PDE5 activity and/or NO related endothelial dysfunction, in a subject, preferably in a human. Preferably, said disease or disorder is selected from ischemic skin ulcers, digital ulcers (DU) in systemic sclerosis, diabetic foot ulcer, leg ulcer, ischemic arterial ulcers, livedoid vasculopathy, Martorell hypertensive ischemic leg ulcer, thromboangiititis obliteragans (Buerger's disease), sickle cell leg ulcer, all of the foregoing preferably for wound healing, further preferably for chronic wound healing. Very preferred, said disease or disorder is systemic sclerosis, and wherein preferably said disease or disorder is digital ulcers in systemic sclerosis.

In another aspect, the present invention provides the topical composition of the present invention for use in a method of topically treating a disease or disorder in a subject, preferably in a human, wherein said disease or disorder is selected from ischemic skin ulcers, digital ulcers (DU) in systemic sclerosis, diabetic foot ulcer, leg ulcer, ischemic arterial ulcers, livedoid vasculopathy, Martorell hypertensive ischemic leg ulcer, thromboangiititis obliteragans (Buerger's disease), sickle cell leg ulcer, all of the foregoing preferably for wound healing, further preferably for chronic wound healing, and wherein preferably said disease or disorder is systemic sclerosis, and again further preferably said disease or disorder is digital ulcers in systemic sclerosis.

In a further aspect, the invention provides a kit comprising:
(i) a first kit component comprising the topical composition of the present invention; and
(ii) a second kit component comprising a patch, a tape, a dressing, a sheet or a device suitable to maintain contact between said topical composition and the skin of a subject, preferably the skin of a human.

Disclosed, but not claimed, is a method of preparing the topical composition of the present invention, wherein said method comprises dissolving a compound of formula I in a solvent, wherein preferably said solvent comprises PEG400, and further preferably wherein said solvent comprises PEG400 and water.

Further aspects and embodiments of the present invention will be become apparent as this description continues.

### DESCRIPTION OF FIGURES

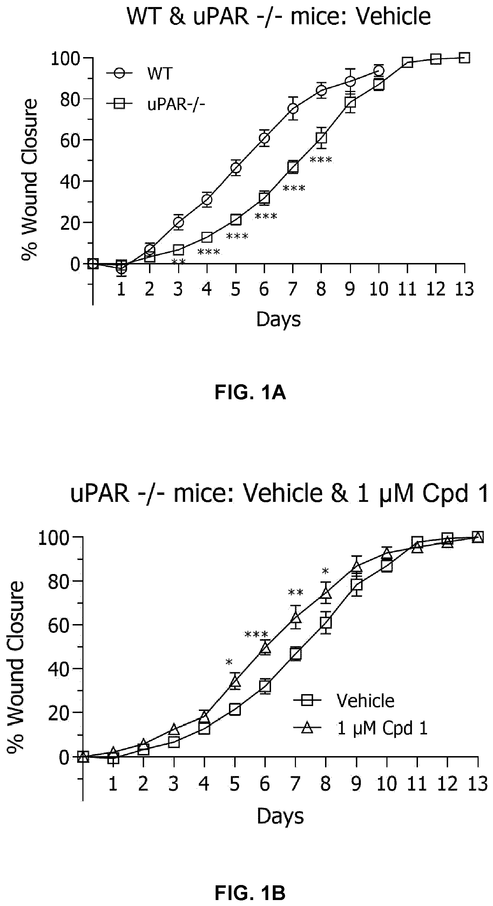

FIG. 1A: Healing of full thickness, excisional, cutaneous wounds (2 wounds / mouse) was followed over 12-14 days from surgery in uPAR -/- mice and wild type (WT) mice (mixed C57BL/6 (75%) ×129 (25%) background). Vehicle Composition **A** was administered once daily, *on wound,* at a volume of 25 µl per wound. Results in the uPAR -/- mice are from initially 12 wounds, 7 mice. Results in the wild type (WT, uPAR+/+) mice are from initially 11 wounds, 11 mice.
FIG. 1B: Healing of full thickness, excisional, cutaneous wounds (2 wounds / mouse) was followed over 12-14 days from surgery in uPAR -/- mice after repeated, once daily, topical, *on wound* administration of a volume of 25 µl per wound of Topical Composition **A2** or Vehicle Composition **A.** Results are from initially 12 wounds, 6 mice (Topical Composition **A2**) and 12 wounds, 7 mice (Vehicle Composition **A**). % Wound closure was calculated as the percent ratio of the difference between the ulcer inner diameter at day k (0≤k≤14) and the ulcer inner diameter at day 0 (immediately after surgery) divided by the ulcer inner diameter at day 0. Results are shown as means ± SEM. Statistical analyses were done by mixed effect analysis, Sidak's multiple comparisons (GraphPad Prism 9.0). *p<0.05, **p<0.01, ***p<0.001 versus wild type (FIG. 1A) or vehicle (FIG. 1B). Only wounds with an intact silicone ring and stitches, used to avoid wound contraction, were included in the analysis that explains less evaluable wounds over time (Table 5). In the vehicle groups, some mice received vehicle on one wound and sham at the other wound which explains that the number of wounds may be less than the 2-fold of the number of mice enrolled.

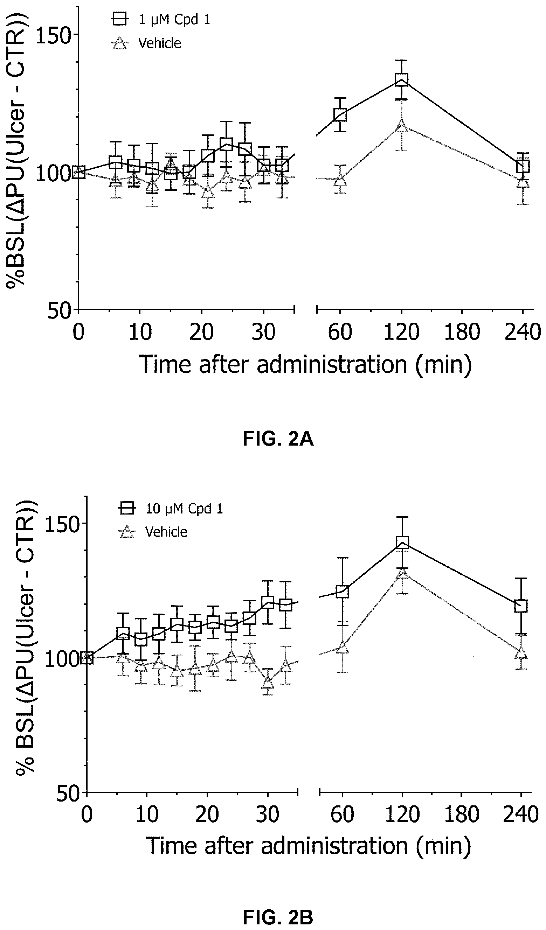

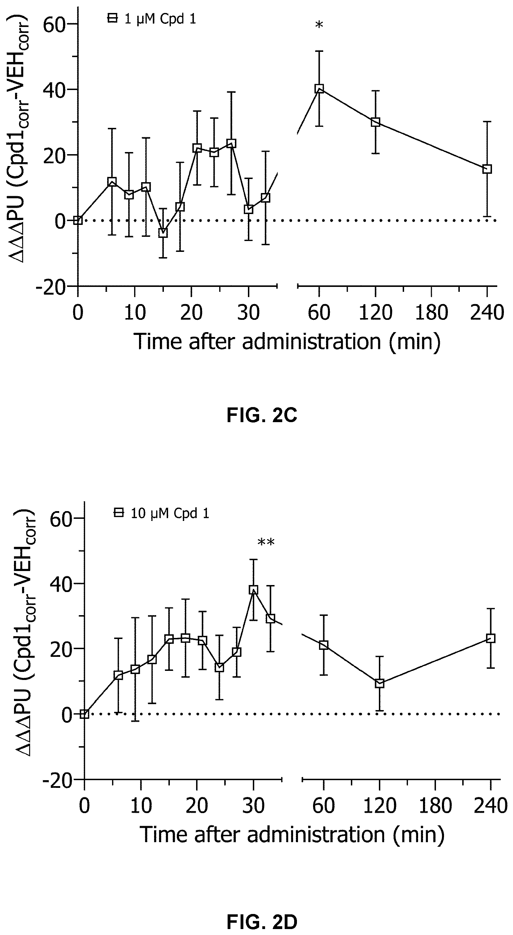

FIG. 2A: For all t, the %BSLₜ(APU(Ulcer-CTR)) for Topical Composition **A2,** Topical Composition **A4** or Vehicle Composition **A** were calculated as the ratio between (i) the differences between the PUt from the ulcer area and the PUt from the corresponding control area (ΔPUₜ = (PUt (Ulcer) - PUt (Control))) at time point t and (ii) time point 0 i.e. ΔPU₀ following the equation %BSLₜ(APU(Ulcer-CTR)) = ΔPUₜ/ΔPU₀ * 100 %. Graphs depict the means ± SEM from %BSLₜ(APU(Ulcer-CTR)) of 8 mice per group for the ulcer area. For any given t (6≤t≤240) there was no statistically significant difference between %BSLₜ(ΔPU(Ulcer-CTR)) for Topical Composition **A2** and Topical Composition **A4** versus Vehicle Composition **A** (2-way ANOVA). Ulcer means the ulcer region of interest (ROI) and CTR means the corresponding control ROI.
FIG. 2B: For all t, ΔΔΔPUₜ(TN53_{corr}-VEH_{corr}) was calculated as the difference between ΔΔPU₍ₜ₋₀₎=ΔPUₜ-ΔPU₀ calculated for (i) Topical Composition **A2** and Topical Composition **A4** and (ii) Vehicle Composition **A** at the ulcer areas. Graphs depict the means ± SEM from ΔΔΔPUₜ(Cpd **1**_{corr}-VEH_{corr}) of 8 mice per group for the ulcer area. *p<0.05, **p<0.01 from baseline (zero) by 2-way ANOVA. A letter 't' in subscript means measurements or calculations at time point t after administration of Topical Composition **A2** and Topical Composition **A4** or Vehicle Composition **A.** Perfusion Units (PU) values in ulcers areas corrected for the corresponding values in the control (CTR) areas.

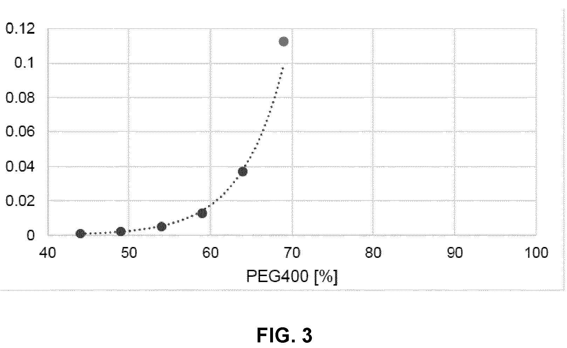

FIG. 3: Solubility of Compound **1** (provided in mg/mL depicted on x-axis) in relation to the concentration of PEG400 at room temperature.

### DETAILED DESCRIPTION OF THE INVENTION

Unless defined otherwise, all technical and scientific terms used herein have the same meanings as commonly understood by one of ordinary skill in the art to which this invention belongs.

The term "about", as used herein shall have the meaning of +/- 10%. For example about 50% shall mean 45% to 55%. Preferably, the term "about", as used herein shall have the meaning of +/- 5%. For example about 50% shall mean 47.5% to 52.5%. In a preferred embodiment, said term "about" refers to any value of a range of and between - 10% and +10% of the value it refers to. In a preferred embodiment, said term "about" refers to any value of a range of and between - 8% and +8% of the value it refers to. In a preferred embodiment, said term "about" refers to any value of and between a range of - 7% and +7% of the value it refers to. In a preferred embodiment, said term "about" refers to any value of and between a range of - 5% to +5% of the value it refers to. In a preferred embodiment, said term "about" refers to any value of and between a range of - 4% and +4% of the value it refers to. In a preferred embodiment, said term "about" refers to any value of and between a range of - 3% and +3% of the value it refers to. In a preferred embodiment, said term "about" refers to any value of and between a range of - 2% and +2% of the value it refers to.

The phrase "between value X and value Y", as used herein, shall refer to include the value X and the value Y and any value in between. For example, the phrase "between 0.01µmol and 50µmol" refers to 0.01µmol and 50µmol and any value in between. The same applies to the phrase "between about value X and about value Y" taking further the variations of the term "about" into account. Analogously, the phrase "from value X to value Y", as used herein, shall refer to include the value X and the value Y and any value in between. For example, the phrase "between 65% and 75%" refers to 65% and 75% and any value in between. The same applies to the phrase "from about value X to about value Y" taking further the variations of the term "about" into account.

The term "% (w/w)"as used herein refers to (mass of the component / total mass of the composition) x 100. By way of example, 70 wt% PEG400 is 70 g PEG400 per 100 g of the composition.

When the terms "a," or "an" are used herein, they mean "at least one" unless indicated otherwise.

As used herein, the term "topically" and "topical" refers to application of the composition of the present invention to the surface of the skin of a subject, preferably of a human, and thus, the term "topical administration", as used herein, refers to administration to the skin of a subject, preferably of a human, preferably to on wound administration.

As used herein the term "topical composition" refers to a composition that is suitable for topical administration and may be applied to skin of a subject, preferably of a human.

As used herein, the terms "treatment", "treat", "treated" or "treating" refer to prophylaxis and/or therapy. In one embodiment, the terms "treatment", "treat", "treated" or "treating" refer to a therapeutic treatment. In another embodiment, the terms "treatment", "treat", "treated" or "treating" refer to a prophylactic treatment. Preferably, beneficial or desired clinical results of said treatment include, but are not limited to, alleviation of symptoms, diminishment of extent of disease or disorder, stabilized (*i.e*., not worsening) state of disease or disorder, delay or slowing of disease or disorder progression, amelioration or palliation of the disease or disorder state.

As used herein, the term "effective amount" refers to an amount necessary or sufficient to realize a desired biologic effect. Preferably, the term "effective amount" refers to an amount of a compound of formula **I** of the present invention that (i) treats or prevents the particular disease or disorder, (ii) attenuates, ameliorates, or eliminates one or more symptoms of the particular disease or disorder, or (iii) prevents or delays the onset of one or more symptoms of the particular disease or disorder, described herein. An effective amount of the inventive compound of formula **I,** or said topical composition or said pharmaceutical composition, would be the amount that achieves this selected result, and such an amount could be determined as a matter of routine by a person skilled in the art. Further preferably, the term "effective amount", as used herein, refers to an amount necessary or sufficient to be effective to activation of soluble guanylyl cyclase (sGC) and/or increase the inhibition of PDE5. The effective amount can vary depending on the particular composition being applied and the size of the subject. One of ordinary skill in the art can empirically determine the effective amount of a particular the inventive compound of formula **I,** or said topical composition or said pharmaceutical composition of the present invention without necessitating undue experimentation.

The term "mammal", as used herein, includes, but is not limited to, humans, mice, rats, guinea pigs, monkeys, dogs, cats, horses, cows, pigs, and sheep. The term "mammal", as used herein, preferably refers to humans. The term "subject", as used herein, includes, but is not limited to, humans and mammals. The term "subject", as used herein, preferably refers to humans.

The term "kit" as used herein means that the components comprised in said kit are provided physically separable and distinguishable from one another as different components but are provided or sold together for the purpose of being administered, or used, together.

Surprisingly, the inventors were able to provide inventive topical compositions, in particular inventive topical compositions comprising Compound **1** despite the shown de-facto non-solubility of the inventive compounds of formula **I,** in particular of Compound **1** in physiological aqueous phosphate buffer (pH 7.5). Moreover, the inventive topical compositions showed efficacy and improved wound healing in uPAR deficient mice, a disease-relevant animal model of systemic sclerosis having cutaneous ulcers, at surprisingly very low concentrations of the inventive compounds of formula **I**, in particular of Compound **1.**

Thus, in a first aspect, the present invention provides a topical composition as claimed comprising a compound of formula **I,** preferably an effective amount of a compound of formula **I,** or a pharmaceutically acceptable salt thereof 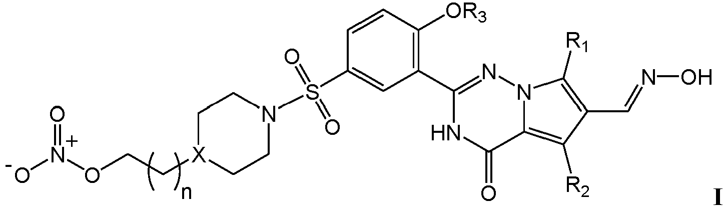 wherein
R₁ is ethyl or propyl, preferably propyl;
R₂ is methyl or ethyl, preferably methyl;
R₃ is ethyl or propyl, preferably propyl;
X is N or CH, preferably CH;
n is 1 or 2, preferably n=1.

In another aspect, the present invention provides a topical composition as claimed comprising
(a) a compound of formula **I,** preferably an effective amount of a compound of formula **I,** or a pharmaceutically acceptable salt thereof 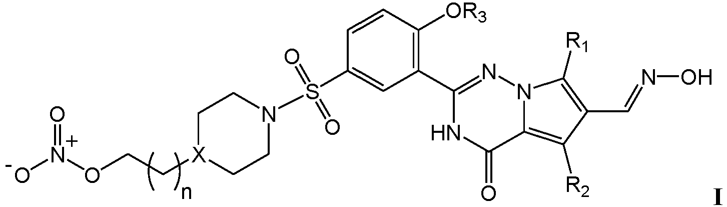 wherein
   R₁ is ethyl or propyl, preferably propyl;
   R₂ is methyl or ethyl, preferably methyl;
   R₃ is ethyl or propyl, preferably propyl;
   X is N or CH, preferably CH;
   n is 1 or 2, preferably n=1;
(b) a solvent; and
(c) optionally one or more other pharmaceutically acceptable excipients.

Compounds of formula **I** comprised in the topical composition of the present invention include pharmaceutically acceptable salts of said compounds. In particular, the term "pharmaceutically acceptable salt" as used herein, refers to pharmaceutically acceptable organic or inorganic salts of a compound of the present invention, in particular acid addition salts. Exemplary salts include, but are not limited to, salts of physiologically acceptable mineral acids, such as hydrochloric acid, sulfuric acid, nitric acid and phosphoric acid, or salts of organic acids, such as methane-sulfonic acid, p-toluenesulfonic acid, lactic acid, malic acid, tartaric acid, acetic acid, trifluoroacetic acid, citric acid, succinic acid, fumaric acid, maleic acid and salicylic acid. Further examples of pharmacologically acceptable salts of the compounds of formula **I** are alkali metal and alkaline earth metal salts such as, for example, sodium, potassium, lithium, calcium or magnesium salts, ammonium salts or salts of organic bases such as, for example, methylamine, dimethylamine, triethylamine, piperidine, ethylenediamine, lysine, choline hydroxide, meglumine, morpholine or arginine salts. Further examples of pharmaceutically acceptable salts of the compounds of formula **I** include the hydrochloride, hydrobromide, sulfate, bisulfate, phosphate, hydrogen phosphate, nitrate, acetate, benzoate, succinate, fumarate, maleate, lactate, citrate, benzenesulphonate, p-toluenesulphonate or the like.

In one embodiment, said R₁ is propyl. In one embodiment, said R₁ is ethyl. In one embodiment, said R₂ is methyl. In one embodiment, said R₂ is ethyl. In one embodiment, said R₃ is propyl. In one embodiment, said R₃ is ethyl. In one embodiment, said X is N. In one embodiment, said X is CH. In one embodiment, said n is 1. In one embodiment, said n is 2.

In one embodiment, said compound of formula **I** is a compound selected from the group consisting of 2-[1-(3-{6-[(1E)-(hydroxyimino)methyl]-5-methyl-4-oxo-7-propyl-3H,4H-pyrrolo[2,1-f][1,2,4]triazin-2-yl}-4-propoxybenzenesulfonyl)piperidin-4-yl]ethyl nitrate (Compound **1**); (E)-2-(2-ethoxy-5-((4-(2-hydroxyethyl)piperazin-1-yl)sulfonyl)phenyl)-5-ethyl-4-oxo-7-propyl-3,4-dihydropyrrolo[2,1-f][1,2,4]triazine-6-carbaldehyde oxime (Compound **2**); (E)-2-(4-((4-ethoxy-3-(6-((hydroxyimino)methyl)-5-methyl-4-oxo-7-propyl-3,4-dihydropyrrolo[2,1-f][1,2,4]triazin-2-yl)phenyl)sulfonyl)piperazin-1-yl)ethyl nitrate (Compound **3**); (*E*)-2-(4-((4-ethoxy-3-(5-ethyl-6-((hydroxyimino)methyl)-4-oxo-7-propyl-3,4-dihydropyrrolo[2,1-f][1,2,4]triazin-2-yl)phenyl)sulfonyl)piperazin-1-yl)ethyl nitrate (Compound **4**); (*E*)-2-(1-((4-ethoxy-3-(6-((hydroxyimino)methyl)-5-methyl-4-oxo-7-propyl-3,4-dihydropyrrolo[2,1-f][1,2,4]triazin-2-yl)phenyl)sulfonyl)piperidin-4-yl)ethyl nitrate (Compound **5**); and (E)-3-(4-((4-ethoxy-3-(6-((hydroxyimino)methyl)-5-methyl-4-oxo-7-propyl-3,4-dihydropyrrolo[2,1-f][1,2,4]triazin-2-yl)phenyl)sulfonyl)piperazin-1-yl)propyl nitrate (Compound **6**).

In a preferred embodiment, said R₁ is propyl, R₂ is methyl, R₃ is propyl, X is CH and n=1. Thus, in a very preferred embodiment, said compound of formula I is 2-[1-(3-{6-[(1E)-(hydroxyimino)methyl]-5-methyl-4-oxo-7-propyl-3H,4H-pyrrolo[2,1-f][1,2,4]triazin-2-yl}-4-propoxybenzenesulfonyl)piperidin-4-yl]ethyl nitrate (Compound **1**). In a preferred embodiment, said compound of formula I is Compound **1**

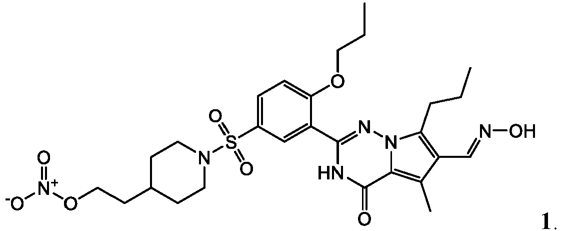

In a very preferred embodiment, said compound of formula **I** is Compound **1**

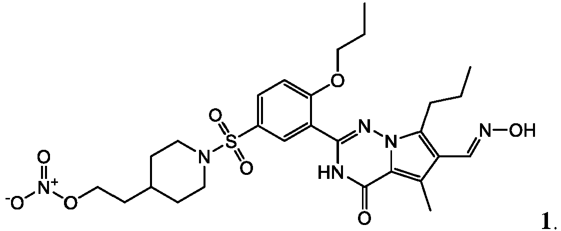

In a preferred embodiment, said compound of formula **I,** preferably said Compound **1,** is present from about 0.000006% (w/w) to about 0.03% (w/w), preferably from about 0.00006% (w/w) to about 0.01% (w/w), more preferably from about 0.0001% (w/w) to about 0.01% (w/w), based on the total weight of said topical composition.

In a further preferred embodiment, said compound of formula **I** is Compound **1,** and said Compound **1** is present from about 0.000006% (w/w) to about 0.03% (w/w), preferably from about 0.00006% (w/w) to about 0.01% (w/w), more preferably from about 0.0001% (w/w) to about 0.01% (w/w), based on the total weight of said topical composition.

In a further preferred embodiment, said compound of formula **I,** preferably said Compound **1,** is present from about 0.0001% (w/w) to about 0.01% (w/w) based on the total weight of said topical composition. In a further preferred embodiment, said compound of formula **I,** preferably said Compound **1,** is present from about 0.00018% (w/w) to about 0.006% (w/w) based on the total weight of said topical composition. In a further preferred embodiment, said compound of formula **I,** preferably said Compound **1,** is present from about 0.0004% (w/w) to about 0.002% (w/w) based on the total weight of said topical composition. In a further preferred embodiment, said compound of formula **I,** preferably said Compound **1,** is present from about 0.0005% (w/w) to about 0.001% (w/w) based on the total weight of said topical composition. In a further preferred embodiment, said compound of formula **I,** preferably said Compound **1,** is present from about 0.0006% (w/w) based on the total weight of said topical composition. In a further preferred embodiment, said compound of formula **I,** preferably said Compound **1,** is present from about 0.0006% (w/w) to about 0.001% (w/w) based on the total weight of said topical composition.

In a further preferred embodiment, said compound of formula **I** is Compound **1,** and said Compound **1** is present from about 0.0001% (w/w) to about 0.01% (w/w) based on the total weight of said topical composition. In a further preferred embodiment, said compound of formula **I** is Compound **1,** and said Compound **1** is present from about 0.00018% (w/w) to about 0.006% (w/w) based on the total weight of said topical composition. In a further preferred embodiment, said compound of formula **I** is Compound **1,** and said Compound **1** is present from about 0.0004% (w/w) to about 0.002% (w/w) based on the total weight of said topical composition. In a further preferred embodiment, said compound of formula **I** is Compound **1,** and said Compound **1** is present from about 0.0005% (w/w) to about 0.001% (w/w) based on the total weight of said topical composition. In a further preferred embodiment, said compound of formula **I** is Compound **1,** and said Compound **1** is present from about 0.0006% (w/w) based on the total weight of said topical composition. In a further preferred embodiment, said compound of formula **I** is Compound **1,** and said Compound **1** is present from about 0.0006% (w/w) to about 0.001% (w/w) based on the total weight of said topical composition.

In a further preferred embodiment, said topical composition comprises said compound of formula **I,** preferably said Compound **1,** in an amount between about 0.01µmol and about 50µmol, preferably between about 0.1µmol and about 20µmol, further preferably between about 0.2µmol and about 15µmol.

In a further preferred embodiment, said topical composition comprises said compound of formula **I,** preferably said Compound **1,** in a concentration between about 0.1µM and about 535µM, preferably between about 1µM and about 214µM, further preferably between about 2µM and about 160µM.

In a further preferred embodiment, said compound of formula **I** is Compound **1,** and said topical composition comprises said Compound **1** in an amount between about 0.01µmol and about 50µmol, preferably between about 0.1µmol and about 20µmol, further preferably between about 0.2µmol and about 15µmol.

In a further preferred embodiment, said compound of formula **I** is Compound **1,** and said topical composition comprises said Compound **1** in a concentration between about 0.1µM and about 535µM, preferably between about 1µM and about 214µM, further preferably between about 2µM and about 160µM.

In a further preferred embodiment, said topical composition comprises said compound of formula **I,** preferably said Compound **1,** in an amount between about 0.2µmol and about 15µmol. In a further preferred embodiment, said topical composition comprises said compound of formula **I,** preferably said Compound **1,** in an amount between about 0.3µmol and about 12µmol. In a further preferred embodiment, said topical composition comprises said compound of formula **I,** preferably said Compound **1,** in an amount between about 0.3µmol and about 10µmol. In a further preferred embodiment, said topical composition comprises said compound of formula **I,** preferably said Compound **1,** in an amount between about 0.3µmol and about 5µmol. In a further preferred embodiment, said topical composition comprises said compound of formula **I,** preferably said Compound **1,** in an amount between about 0.5µmol and about 10µmol. In a further preferred embodiment, said topical composition comprises said compound of formula **I,** preferably said Compound **1,** in an amount between about 0.5µmol and about 5µmol. In a further preferred embodiment, said topical composition comprises said compound of formula **I,** preferably said Compound **1,** in an amount between about 0.7µmol and about 5µmol. In a further preferred embodiment, said topical composition comprises said compound of formula **I,** preferably said Compound **1,** in an amount between about 0.8µmol and about 2µmol. In a further preferred embodiment, said topical composition comprises said compound of formula **I,** preferably said Compound **1,** in an amount of about 1µmol.

In a further preferred embodiment, said topical composition comprises said compound of formula **I,** preferably said Compound **1,** in a concentration between about 2µM and about 160µM. In a further preferred embodiment, said topical composition comprises said compound of formula **I,** preferably said Compound **1,** in a concentration between about 3µM and about 130µM. In a further preferred embodiment, said topical composition comprises said compound of formula **I,** preferably said Compound **1,** in a concentration between about 3µM and about 110µM. In a further preferred embodiment, said topical composition comprises said compound of formula **I,** preferably said Compound **1,** in a concentration between about 3µM and about 55µM. In a further preferred embodiment, said topical composition comprises said compound of formula **I,** preferably said Compound **1,** in a concentration between about 5µM and about 110µM. In a further preferred embodiment, said topical composition comprises said compound of formula **I,** preferably said Compound **1,** in a concentration between about 5µM and about 55µM. In a further preferred embodiment, said topical composition comprises said compound of formula **I,** preferably said Compound **1,** in a concentration between about 7µM and about 55µM. In a further preferred embodiment, said topical composition comprises said compound of formula **I,** preferably said Compound **1,** in a concentration between about 8µM and about 22µM. In a further preferred embodiment, said topical composition comprises said compound of formula **I,** preferably said Compound **1,** in a concentration between about 10µM and about 12µM. In a further preferred embodiment said topical composition comprises said compound of formula **I,** preferably said Compound **1,** in a concentration of about 11µM.

In a further preferred embodiment, said compound of formula I is Compound **1,** and said topical composition comprises said Compound **1** in an amount between about 0.2µmol and about 15µmol. In a further preferred embodiment, said compound of formula **I** is Compound **1,** and said topical composition comprises said Compound **1** in an amount between about 0.3µmol and about 12µmol. In a further preferred embodiment, said compound of formula **I** is Compound 1, and said topical composition comprises said Compound **1** in an amount between about 0.3µmol and about 10µmol. In a further preferred embodiment, said compound of formula **I** is Compound **1,** and said topical composition comprises said Compound **1** in an amount between about 0.3µmol and about 5µmol. In a further preferred embodiment, said compound of formula **I** is Compound **1,** and said topical composition comprises said Compound **1** in an amount between about 0.5µmol and about 10µmol. In a further preferred embodiment, said compound of formula **I** is Compound **1,** and said topical composition comprises said Compound **1** in an amount between about 0.5µmol and about 5µmol. In a further preferred embodiment, said compound of formula **I** is Compound **1,** and said topical composition comprises said Compound **1** in an amount between about 0.7µmol and about 5µmol. In a further preferred embodiment, said compound of formula **I** is Compound **1,** and said topical composition comprises said Compound **1** in an amount between about 0.8µmol and about 2µmol. In a further preferred embodiment, said compound of formula **I** is Compound **1,** and said topical composition comprises said Compound **1** in an amount of about 1µmol.

In a further preferred embodiment, said compound of formula I is Compound **1,** and said topical composition comprises said Compound **1** in a concentration between about 2µM and about 165µM. In a further preferred embodiment, said compound of formula **I** is Compound **1,** and said topical composition comprises said Compound **1** in a concentration between about 3µM and about 130µM. In a further preferred embodiment, said compound of formula **I** is Compound **1,** and said topical composition comprises said Compound **1** in a concentration between about 3 µM and about 110µM. In a further preferred embodiment, said compound of formula **I** is Compound **1,** and said topical composition comprises said Compound **1** in a concentration between about 3µM and about 55µM. In a further preferred embodiment, said compound of formula **I** is Compound **1,** and said topical composition comprises said Compound **1** in a concentration between about 5µM and about 110µM. In a further preferred embodiment, said compound of formula **I** is Compound **1,** and said topical composition comprises said Compound **1** in a concentration between about 5µM and about 55µM. In a further preferred embodiment, said compound of formula **I** is Compound **1,** and said topical composition comprises said Compound **1** in a concentration between about 7µM and about 55µM. In a further preferred embodiment, said compound of formula **I** is Compound **1,** and said topical composition comprises said Compound **1** in a concentration between about 8µM and about 22µM. In a further preferred embodiment, said compound of formula **I** is Compound **1,** and said topical composition comprises said Compound **1** in a concentration between about 10µM and about 12µM. In a further preferred embodiment, said compound of formula **I** is Compound **1,** and said topical composition comprises said Compound **1** in a concentration of about 11µM.

In some embodiments, said topical composition can be formulated to different dosage forms, for example, a solution, a suspension, a cream, an ointment, a lotion, a paste, an emulsion, a foam and a gel.

In a preferred embodiment, said topical composition is formulated as a solution or a gel. In a preferred embodiment, said topical composition is in the form of a gel, preferably of an aqueous gel. In a preferred embodiment, said topical composition is a gel, preferably an aqueous gel.

In a preferred embodiment, said topical composition is a liquid topical composition. In a further preferred embodiment, said topical composition is a liquid topical composition, wherein said liquid topical composition is formulated as a solution or a gel. In a further preferred embodiment, said topical composition is a liquid topical composition, wherein said liquid topical composition is formulated as a solution. In a further preferred embodiment, said topical composition is a liquid topical composition, wherein said liquid topical composition is formulated as a gel. In a further preferred embodiment, said topical composition is a liquid topical composition, wherein said liquid topical composition is formulated as an aqueous solution. In a further preferred embodiment, said topical composition is a liquid topical composition, wherein said liquid topical composition is formulated as an aqueous solution, and wherein said solvent comprises water. In a further preferred embodiment, said topical composition is a liquid topical composition, wherein said liquid topical composition is formulated as an aqueous gel. In a further preferred embodiment, said topical composition is a liquid topical composition, wherein said liquid topical composition is formulated as an aqueous gel, and wherein said solvent comprises water.

In a further preferred embodiment, said topical composition is a liquid topical composition, wherein said liquid topical composition is in the form of a solution or a gel. In a further preferred embodiment, said topical composition is a liquid topical composition, wherein said liquid topical composition is in the form of a solution. In a further preferred embodiment, said topical composition is a liquid topical composition, wherein said liquid topical composition is in the form of a gel. In a further preferred embodiment, said topical composition is a liquid topical composition, wherein said liquid topical composition is in the form of an aqueous solution. In a further preferred embodiment, said topical composition is a liquid topical composition, wherein said liquid topical composition is in the form of an aqueous solution, and wherein said solvent comprises water. In a further preferred embodiment, said topical composition is a liquid topical composition, wherein said liquid topical composition is in the form of an aqueous gel. In a further preferred embodiment, said topical composition is a liquid topical composition, wherein said liquid topical composition is in the form of an aqueous gel, and wherein said solvent comprises water.

In a further preferred embodiment, said topical composition is a liquid topical composition, and wherein preferably said liquid topical composition is in the form of an aqueous gel.

According to the claims, said topical composition comprises PEG 400. In a preferred embodiment, said topical composition is in the form of a gel, and wherein said topical composition comprises PEG 400.

According to the claims, said solvent of said topical composition comprises PEG 400, and wherein the amount of said PEG 400 is from about 60% to about 75 % (w/w), preferably from about 65% to about 75 % (w/w), and more preferably from about 68 % to about 72 % (w/w) based on the total weight of the topical composition. In a further preferred embodiment, the amount of said PEG 400 is from about 60% to about 75 % (w/w) based on the total weight of the topical composition. In a further preferred embodiment, the amount of said PEG 400 is from about 65% to about 75 % (w/w) based on the total weight of the topical composition. In a further preferred embodiment, the amount of said PEG 400 is from about 68 % to about 72 % (w/w) based on the total weight of the topical composition. In a further preferred embodiment, the amount of said PEG 400 is from 60% to 75 % (w/w) based on the total weight of the topical composition. In a further preferred embodiment, the amount of said PEG 400 is about 65% to 75 % (w/w) based on the total weight of the topical composition. In a further preferred embodiment, the amount of said PEG 400 is from 68 % to 72 % (w/w) based on the total weight of the topical composition. In a preferred embodiment, said solvent comprises PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition.

According to the claims, said solvent of said topical composition comprises PEG 400. In a preferred embodiment, said topical composition is in the form of a gel, preferably of an aqueous gel, and wherein said solvent comprises PEG 400.

In a further preferred embodiment, said solvent comprises PEG 400.

In a preferred embodiment, said topical composition comprises PEG 400 and water. In a very preferred embodiment, said topical composition is in the form of an aqueous gel, wherein said solvent comprises PEG 400 and water.

According to the claims, said solvent of said topical composition comprises PEG 400, and wherein the amount of said PEG 400 is from about 60% to about 75 % (w/w), preferably from about 65% to about 75 % (w/w), and more preferably from about 68 % to about 72 % (w/w) based on the total weight of the topical composition. In a preferred embodiment, said topical composition is in the form of a gel, preferably of an aqueous gel, and wherein said solvent comprises PEG 400, and wherein the amount of said PEG 400 is from about 60% to about 75 % (w/w), preferably from about 65% to about 75 % (w/w), and more preferably from about 68 % to about 72 % (w/w) based on the total weight of the topical composition.

In a preferred embodiment, said topical composition comprises PEG 400 and water. In a very preferred embodiment, said topical composition is in the form of an aqueous gel, wherein said solvent comprises PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition.

In a preferred embodiment, said topical composition comprises an aqueous buffer, preferably an aqueous phosphate buffer. In a preferred embodiment, said topical composition is in the form of a gel, preferably in form of an aqueous gel, and wherein said topical composition comprises an aqueous buffer, preferably an aqueous phosphate buffer. In a preferred embodiment, said topical composition is in the form of an aqueous gel, and wherein said topical composition comprises an aqueous phosphate buffer.

In a preferred embodiment, said topical composition comprises PEG 400 and water. In a very preferred embodiment, said topical composition is in the form of an aqueous gel, wherein said solvent comprises PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition, and wherein said topical composition comprises an aqueous buffer, preferably an aqueous phosphate buffer. In a very preferred embodiment, said topical composition is in the form of an aqueous gel, wherein said solvent comprises PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition, and wherein said topical composition comprises an aqueous phosphate buffer.

In a preferred embodiment, said topical composition comprises an aqueous buffer, preferably an aqueous phosphate buffer, of a pH of about 6.5 to about 7.5, further preferably an aqueous buffer, preferably an aqueous phosphate buffer, of a pH of about 6.5 to about 7.0, even more preferably an aqueous buffer, preferably an aqueous phosphate buffer, of a pH of about 6.7. In a preferred embodiment, said topical composition is in the form of a gel, preferably in form of an aqueous gel, and wherein said topical composition comprises an aqueous phosphate buffer, wherein preferably said topical composition comprises an aqueous phosphate buffer of a pH of about 6.5 to about 7.5, further preferably an aqueous phosphate buffer of a pH of about 6.5 to about 7.0, even more preferably an aqueous phosphate buffer of a pH of about 6.7.

In a preferred embodiment, said aqueous buffer, preferably said aqueous phosphate buffer, has a molarity of about 10 to about 15 mM, preferably of about 12mM. In a preferred embodiment, said aqueous buffer is an aqueous phosphate buffer, wherein said aqueous phosphate buffer has a molarity of about 10 to about 15 mM, preferably of about 12mM.

In a preferred embodiment, said aqueous buffer, preferably said aqueous phosphate buffer, is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition. In a preferred embodiment, said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition.

In a preferred embodiment, said topical composition comprises an aqueous phosphate buffer, preferably an aqueous phosphate buffer of a pH of about 6.5 to about 7.5, further preferably an aqueous phosphate buffer of a pH of about 6.5 to about 7.0, even more preferably an aqueous phosphate buffer of a pH of about 6.7, and wherein said aqueous phosphate buffer has a molarity of about 10 to about 15 mM, preferably of about 12mM.

In a preferred embodiment, said topical composition comprises an aqueous phosphate buffer, preferably an aqueous phosphate buffer of a pH of about 6.5 to about 7.5, further preferably an aqueous phosphate buffer of a pH of about 6.5 to about 7.0, even more preferably an aqueous phosphate buffer of a pH of about 6.7, and wherein said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition.

In a preferred embodiment, said topical composition comprises PEG 400 and water. In a very preferred embodiment, said topical composition is in the form of an aqueous gel, wherein said solvent comprises PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition, and wherein said topical composition comprises an aqueous buffer, preferably an aqueous phosphate buffer, wherein said topical composition comprises an aqueous buffer, preferably an aqueous phosphate buffer, of a pH of about 6.5 to about 7.5, preferably an aqueous phosphate buffer of a pH of about 6.5 to about 7.0, and more preferably an aqueous buffer, preferably an aqueous phosphate buffer, of a pH of about 6.7.

In a preferred embodiment, said topical composition comprises PEG 400 and water. In a very preferred embodiment, said topical composition is in the form of an aqueous gel, wherein said solvent comprises PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition, and wherein said topical composition comprises an aqueous phosphate buffer, wherein said topical composition comprises an aqueous phosphate buffer of a pH of about 6.5 to about 7.5, preferably an aqueous phosphate buffer of a pH of about 6.5 to about 7.0, and more preferably an aqueous phosphate buffer of a pH of about 6.7.

In a preferred embodiment, said topical composition is in the form of an aqueous gel, and wherein said solvent comprises PEG 400 and water.

According to the claims, the amount of said PEG 400 is from about 60% to about 75 % (w/w), preferably from about 65% to about 75 % (w/w), and more preferably from about 68 % to about 72 % (w/w) based on the total weight of the topical composition.

In a preferred embodiment, the solvent is present at a concentration from about 90% w/w to about 99% w/w, preferably about 95% w/w to about 99% w/w, more preferably about 97% w/w to about 98% w/w, based on the total weight of the topical composition.

In one embodiment, said pharmaceutically acceptable excipient is selected from the group consisting of a thickening agent, a stabilizer, an antioxidant, a chelating agent, an oily material, an emulsifier, a penetration enhancer, a pH adjusting agent, a preservative, an antimicrobial agent, an opacifier, a fragrance, a colorant, a gelling agent, a moisturizer, a surfactant, and a combination thereof. In one embodiment, said pharmaceutically acceptable excipient is selected from the group consisting of a thickening agent, a stabilizer, an antioxidant, a pH adjusting agent, a preservative, an antimicrobial agent, and a combination thereof.

In one embodiment, said topical composition comprises one or more other pharmaceutically acceptable excipients, and wherein said pharmaceutically acceptable excipient is selected from the group consisting of a thickening agent, a stabilizer, an antioxidant, a chelating agent, an oily material, an emulsifier, a penetration enhancer, a pH adjusting agent, a preservative, an antimicrobial agent, an opacifier, a fragrance, a colorant, a gelling agent, a moisturizer, a surfactant, and a combination thereof. In one embodiment, said topical composition comprises one or more other pharmaceutically acceptable excipients, and said pharmaceutically acceptable excipient is selected from the group consisting of a thickening agent, a stabilizer, an antioxidant, a pH adjusting agent, a preservative, an antimicrobial agent, and a combination thereof.

The pharmaceutically acceptable excipients used in the topical composition of the present invention can act in more than one way. For example, a thickening agent can also function as a gelling agent.

In a preferred embodiment, said topical composition comprises one or more other pharmaceutically acceptable excipients, and one of said pharmaceutically acceptable excipients is a thickening agent. In a preferred embodiment, said topical composition comprises a thickening agent.

Thickening agents are known to the skilled person in the art and includes a cellulose derivative, polyvinylpyrrolidone, a carbomer polymer, carbomer derivative, maltodextrin, polydextrose, dextrates, carboxypolymethylene, polyvinyl alcohol, poloxamers and mixtures thereof. Thickening agents may serve, in particular by way of the final viscosity of the topical composition, to ensure and increase the time of stay of the topical composition, for example, on the fingertip wound of the ulcera during the treatment regime.

In a preferred embodiment, said topical composition comprises one or more other pharmaceutically acceptable excipients, and one of said pharmaceutically acceptable excipients is a thickening agent, wherein said thickening agent is selected from the group consisting of carboxymethyl cellulose, hydroxyethyl cellulose, sodium hyaluronate and polyvinylpyrrolidon. In a preferred embodiment, said topical composition comprises a thickening agent, wherein said thickening agent is selected from the group consisting of carboxymethyl cellulose, hydroxyethyl cellulose, sodium hyaluronate and polyvinylpyrrolidon.

In a preferred embodiment, said topical composition comprises one or more excipient selected from a cellulose derivative, polyvinylpyrrolidone, a carbomer polymer, carbomer derivative, maltodextrin, polydextrose, dextrates, carboxypolymethylene, polyvinyl alcohol, poloxamers or a mixture thereof.

In a preferred embodiment, said topical composition comprises one or more excipient selected from the group consisting of carboxymethyl cellulose, hydroxyethyl cellulose, sodium hyaluronate and polyvinylpyrrolidon. In a preferred embodiment, said topical composition comprises one or more excipient selected from the group consisting of carboxymethyl cellulose, hydroxyethyl cellulose, sodium hyaluronate and polyvinylpyrrolidon.

In a preferred embodiment, said topical composition comprises a thickening agent, wherein preferably said thickening agent is selected from the group consisting of carboxymethyl cellulose, hydroxyethyl cellulose, sodium hyaluronate and polyvinylpyrrolidon.

In a preferred embodiment, said topical composition comprises one or more other pharmaceutically acceptable excipients, and one of said pharmaceutically acceptable excipients is a thickening agent, wherein said thickening agent is hydroxyethyl cellulose. In a preferred embodiment, said topical composition comprises a thickening agent, wherein said thickening agent is hydroxyethyl cellulose.

In a preferred embodiment, said topical composition comprises one or more other pharmaceutically acceptable excipients, and one of said pharmaceutically acceptable excipients is a thickening agent, wherein said thickening agent is sodium hyaluronate. In a preferred embodiment, said topical composition comprises a thickening agent, wherein said thickening agent is sodium hyaluronate.

In a preferred embodiment, said topical composition comprises one or more other pharmaceutically acceptable excipients, and one of said pharmaceutically acceptable excipients is hydroxyethyl cellulose.

In a preferred embodiment, said topical composition comprises one or more other pharmaceutically acceptable excipients, and one of said pharmaceutically acceptable excipients is sodium hyaluronate.

In some embodiments, the thickening agent is present from about 0.01% (w/w) to about 5% (w/w), preferably about 0.05% (w/w) to about 2% (w/w), more preferably about 0.1% (w/w) to about 1% (w/w), based on the total weight of said topical composition.

In a preferred embodiment, said topical composition comprises one or more other pharmaceutically acceptable excipients, and one of said pharmaceutically acceptable excipients is a thickening agent, wherein said thickening agent is hydroxyethyl cellulose, and wherein said hydroxyethyl cellulose is present from about 0.1% (w/w) to about 1% (w/w), preferably about 0.1% (w/w) to about 0.5% (w/w), more preferably about 0.25% (w/w) based on the total weight of said topical composition. In a preferred embodiment, said topical composition comprises a thickening agent, wherein said thickening agent is hydroxyethyl cellulose, and wherein said hydroxyethyl cellulose is present from about 0.1% (w/w) to about 1% (w/w), preferably about 0.1% (w/w) to about 0.5% (w/w), more preferably about 0.25% (w/w) based on the total weight of said topical composition.

In a preferred embodiment, said topical composition comprises one or more other pharmaceutically acceptable excipients, and one of said pharmaceutically acceptable excipients is a thickening agent, wherein said thickening agent is sodium hyaluronate, and wherein said sodium hyaluronate is present from about 0.1% (w/w) to about 1% (w/w), preferably about 0.2% (w/w) to about 0.8% (w/w), more preferably about 0.50% (w/w) based on the total weight of said topical composition. In a preferred embodiment, said topical composition comprises a thickening agent, wherein said thickening agent is sodium hyaluronate, and wherein said sodium hyaluronate is present from about 0.1% (w/w) to about 1% (w/w), preferably about 0.2% (w/w) to about 0.8% (w/w), more preferably about 0.50% (w/w) based on the total weight of said topical composition.

In a preferred embodiment, said topical composition comprises hydroxyethyl cellulose. In a preferred embodiment, said topical composition comprises hydroxyethyl cellulose, and wherein said hydroxyethyl cellulose is present from about 0.1% (w/w) to about 1% (w/w), preferably about 0.1% (w/w) to about 0.5% (w/w), more preferably about 0.25% (w/w) based on the total weight of said topical composition.

In a preferred embodiment, said topical composition comprises sodium hyaluronate. In a preferred embodiment, said topical composition comprises sodium hyaluronate, and wherein said sodium hyaluronate is present from about 0.1% (w/w) to about 1% (w/w), preferably about 0.2% (w/w) to about 0.8% (w/w), more preferably about 0.50% (w/w) based on the total weight of said topical composition.

In a preferred embodiment, said topical composition comprises one or more other pharmaceutically acceptable excipients, and one of said pharmaceutically acceptable excipients is an antioxidant. In a preferred embodiment, said topical composition comprises an antioxidant.

Antioxidants are known to the skilled person in the art and includes, for example, butylated hydroxyanisole, butylated hydroxytoluene, vitamin C, vitamin E, vitamin A, lutein, lycopene, retinyl palmitate, potassium metabisulfite, sodium metabisulfite, sodium thiosulfate pentahydrate, 3,4-dihydroxybenzoic acid, propyl gallate, alpha-lipoic acid, ascorbyl palmitate, sodium pyrosulfite, ubiquinone, selenium, or a combination thereof.

In a preferred embodiment, said topical composition comprises one or more other pharmaceutically acceptable excipients, and one of said pharmaceutically acceptable excipients is an antioxidant, wherein said antioxidant is butylated hydroxytoluene. In a preferred embodiment, said topical composition comprises an antioxidant, wherein said antioxidant is butylated hydroxytoluene.

In a preferred embodiment, said topical composition comprises one or more excipient selected from butylated hydroxyanisole, butylated hydroxytoluene, vitamin C, vitamin E, vitamin A, lutein, lycopene, retinyl palmitate, potassium metabisulfite, sodium metabisulfite, sodium thiosulfate pentahydrate, 3,4-dihydroxybenzoic acid, propyl gallate, alpha-lipoic acid, ascorbyl palmitate, sodium pyrosulfite, ubiquinone, selenium, or a combination thereof. In a preferred embodiment, said topical composition comprises butylated hydroxytoluene.

In a preferred embodiment, said topical composition comprises an antioxidant, wherein preferably said antioxidant is butylated hydroxytoluene.

In some embodiments, the antioxidant is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition.

In a preferred embodiment, said topical composition comprises one or more other pharmaceutically acceptable excipients, and one of said pharmaceutically acceptable excipients is an antioxidant, wherein said antioxidant is butylated hydroxytoluene, and wherein said butylated hydroxytoluene is present from about 0.001% (w/w) to about 0.1% (w/w), preferably from about 0.001% (w/w) to about 0.01% (w/w), more preferably from about 0.002% (w/w) to about 0.008% (w/w), and even more preferably of about 0.006% (w/w) based on the total weight of said topical composition. In a preferred embodiment, said topical composition comprises a an antioxidant, wherein said antioxidant is butylated hydroxytoluene, and wherein said butylated hydroxytoluene is present from about 0.001% (w/w) to about 0.1% (w/w), preferably from about 0.001% (w/w) to about 0.01% (w/w), more preferably from about 0.002% (w/w) to about 0.008% (w/w), and even more preferably of about 0.006% (w/w) based on the total weight of said topical composition.

In a preferred embodiment, said topical composition comprises butylated hydroxytoluene, and wherein said butylated hydroxytoluene is present from about 0.001% (w/w) to about 0.1% (w/w), preferably from about 0.001% (w/w) to about 0.01% (w/w), more preferably from about 0.002% (w/w) to about 0.008% (w/w), and even more preferably of about 0.006% (w/w) based on the total weight of said topical composition.

In a preferred embodiment, said topical composition comprises one or more other pharmaceutically acceptable excipients, and one of said pharmaceutically acceptable excipients is a preservative. In a preferred embodiment, said topical composition comprises a preservative.

Preservatives are known to the skilled person in the art and includes, for example, benzyl alcohol, benzoic acid, phenol, m-cresol, methyl parabene, propyl parabene, or a combination thereof.

In a preferred embodiment, said topical composition comprises one or more other pharmaceutically acceptable excipients, and one of said pharmaceutically acceptable excipients is a preservative, wherein said preservative is benzyl alcohol. In a preferred embodiment, said topical composition comprises a preservative, wherein said preservative is benzyl alcohol.

In a preferred embodiment, said topical composition comprises one or more excipient selected from benzyl alcohol, benzoic acid, phenol, m-cresol, methyl parabene, propyl parabene, or a combination thereof. In a preferred embodiment, said topical composition comprises benzyl alcohol.

In a preferred embodiment, said topical composition comprises a preservative, wherein preferably said preservative is benzyl alcohol.

In some embodiments, the preservative is present from about 0.01% (w/w) to about 5% (w/w), preferably about 0.1% (w/w) to about 5% (w/w), more preferably about 1% (w/w) to about 3% (w/w), based on the total weight of said topical composition.

In a preferred embodiment, said topical composition comprises one or more other pharmaceutically acceptable excipients, and one of said pharmaceutically acceptable excipients is a preservative, wherein said preservative is benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition. In a preferred embodiment, said topical composition comprises a preservative, wherein said preservative is benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said topical composition comprises benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition; and wherein said topical composition comprises
(i) an aqueous buffer, preferably an aqueous phosphate buffer, and further preferably an aqueous buffer, preferably an aqueous phosphate buffer, of a pH of about 6.5 to about 7.5;
(ii) a thickening agent, wherein preferably said thickening agent is selected from the group consisting of carboxymethyl cellulose, hydroxyethyl cellulose, sodium hyaluronate and polyvinylpyrrolidon, and wherein further preferably said thickening agent is hydroxyethyl cellulose or sodium hyaluronate, and more preferably wherein said thickening agent is hydroxyethyl cellulose;
(iii) an antioxidant, wherein preferably said antioxidant is butylated hydroxytoluene; and
(iv) a preservative, wherein preferably said preservative is benzyl alcohol.

In a preferred embodiment, said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition; and wherein said topical composition comprises
(i) an aqueous buffer, preferably an aqueous phosphate buffer, and further preferably an aqueous buffer, preferably an aqueous phosphate buffer, of a pH of about 6.5 to about 7.5;
(ii) carboxymethyl cellulose, hydroxyethyl cellulose, sodium hyaluronate or polyvinylpyrrolidon, preferably hydroxyethyl cellulose or sodium hyaluronate, and more preferably hydroxyethyl cellulose;
(iii) butylated hydroxytoluene; and
(iv) benzyl alcohol.

In a preferred embodiment, said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition; and wherein said topical composition comprises
(i) an aqueous buffer, preferably an aqueous phosphate buffer, and further preferably an aqueous buffer, preferably an aqueous phosphate buffer, of a pH of about 6.5 to about 7.5;
(ii) a thickening agent, wherein said thickening agent is hydroxyethyl cellulose or sodium hyaluronate, preferably wherein said thickening agent is hydroxyethyl cellulose;
(iii) an antioxidant, wherein said antioxidant is butylated hydroxytoluene; and
(iv) a preservative, wherein said preservative is benzyl alcohol.

In a preferred embodiment, said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition; and wherein said topical composition comprises
(i) an aqueous buffer, preferably an aqueous phosphate buffer, and further preferably an aqueous buffer, preferably an aqueous phosphate buffer, of a pH of about 6.5 to about 7.5;
(ii) hydroxyethyl cellulose or sodium hyaluronate, preferably hydroxyethyl cellulose;
(iii) butylated hydroxytoluene; and
(iv) benzyl alcohol.

In a preferred embodiment, said compound of formula **I** is Compound **1,** 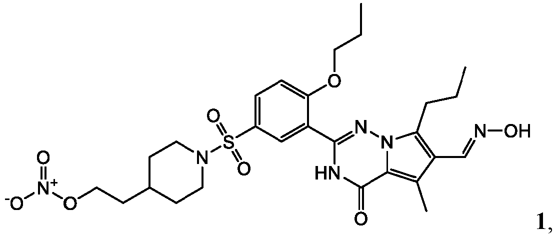 and
wherein said topical composition comprises said Compound **1** in an amount between about 0.7µmol and about 5µmol, preferably in an amount between about 0.8µmol and about 2µmol, and further preferably in an amount of about 1 µmol; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous buffer, preferably an aqueous phosphate buffer, and further preferably an aqueous buffer, preferably an aqueous phosphate buffer, of a pH of about 6.5 to about 7.5, wherein preferably said aqueous buffer, preferably said aqueous phosphate buffer has a molarity of about 10 to about 15 mM, preferably of about 12mM, and wherein said aqueous buffer, preferably said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) a thickening agent, wherein said thickening agent is hydroxyethyl cellulose or sodium hyaluronate, preferably wherein said thickening agent is hydroxyethyl cellulose, and wherein preferably the thickening agent is present from about 0.01% (w/w) to about 5% (w/w), preferably about 0.05% (w/w) to about 2% (w/w), more preferably about 0.1% (w/w) to about 1% (w/w), based on the total weight of said topical composition;
   (iii) an antioxidant, wherein said antioxidant is butylated hydroxytoluene, and wherein preferably said antioxidant is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
   (iv) a preservative, wherein said preservative is benzyl alcohol, and wherein preferably said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 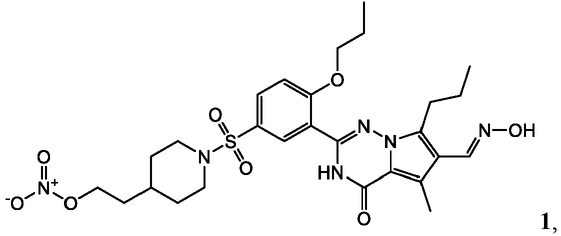 and
wherein said topical composition comprises said Compound **1** in a concentration between about 7µM and about 55µM, preferably in a concentration between about 8µM and about 22µM, further preferably in a concentration between about 10µM and about 12µM, and again further preferably in a concentration of about 11µM; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous buffer, preferably an aqueous phosphate buffer, and further preferably an aqueous buffer, preferably an aqueous phosphate buffer, of a pH of about 6.5 to about 7.5, wherein preferably said aqueous buffer, preferably said aqueous phosphate buffer has a molarity of about 10 to about 15 mM, preferably of about 12mM, and wherein said aqueous buffer, preferably said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) a thickening agent, wherein said thickening agent is hydroxyethyl cellulose or sodium hyaluronate, preferably wherein said thickening agent is hydroxyethyl cellulose, and wherein preferably the thickening agent is present from about 0.01% (w/w) to about 5% (w/w), preferably about 0.05% (w/w) to about 2% (w/w), more preferably about 0.1% (w/w) to about 1% (w/w), based on the total weight of said topical composition;
   (iii) an antioxidant, wherein said antioxidant is butylated hydroxytoluene, and wherein preferably said antioxidant is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
   (iv) a preservative, wherein said preservative is benzyl alcohol, and wherein preferably said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 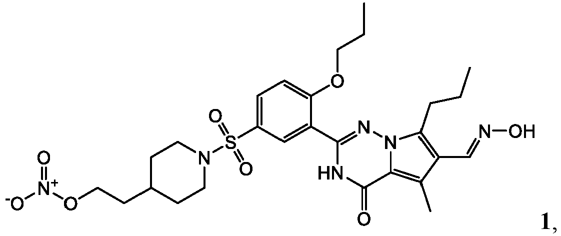 and
wherein said topical composition comprises said Compound **1** in an amount between about 0.7µmol and about 5µmol, preferably in an amount between about 0.8µmol and about 2µmol, and further preferably in an amount of about 1µmol; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous buffer, preferably an aqueous phosphate buffer, and further preferably an aqueous buffer, preferably an aqueous phosphate buffer, of a pH of about 6.5 to about 7.5, wherein preferably said aqueous buffer, preferably said aqueous phosphate buffer has a molarity of about 10 to about 15 mM, preferably of about 12mM, and wherein said aqueous buffer, preferably said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) hydroxyethyl cellulose or sodium hyaluronate, preferably hydroxyethyl cellulose, and wherein preferably hydroxyethyl cellulose or sodium hyaluronate is present from about 0.01% (w/w) to about 5% (w/w), preferably about 0.05% (w/w) to about 2% (w/w), more preferably about 0.1% (w/w) to about 1% (w/w), based on the total weight of said topical composition;
   (iii) butylated hydroxytoluene, and wherein preferably butylated hydroxytoluene is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
   (iv) benzyl alcohol, and wherein preferably said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 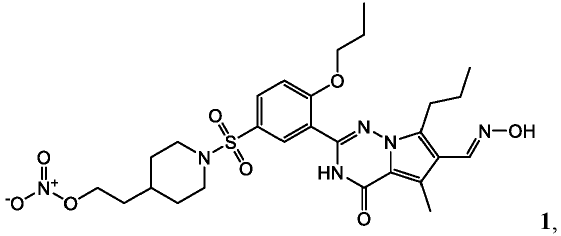 and
wherein said topical composition comprises said Compound **1** in a concentration between about 7µM and about 55µM, preferably in a concentration between about 8µM and about 22µM, further preferably in a concentration between about 10µM and about 12µM, and again further preferably in a concentration of about 11µM; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous buffer, preferably an aqueous phosphate buffer, and further preferably an aqueous buffer, preferably an aqueous phosphate buffer, of a pH of about 6.5 to about 7.5, wherein preferably said aqueous buffer, preferably said aqueous phosphate buffer has a molarity of about 10 to about 15 mM, preferably of about 12mM, and wherein said aqueous buffer, preferably said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) hydroxyethyl cellulose or sodium hyaluronate, preferably hydroxyethyl cellulose, and wherein preferably hydroxyethyl cellulose or sodium hyaluronate is present from about 0.01% (w/w) to about 5% (w/w), preferably about 0.05% (w/w) to about 2% (w/w), more preferably about 0.1% (w/w) to about 1% (w/w), based on the total weight of said topical composition;
   (iii) butylated hydroxytoluene, and wherein preferably butylated hydroxytoluene is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
   (iv) benzyl alcohol, and wherein preferably said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 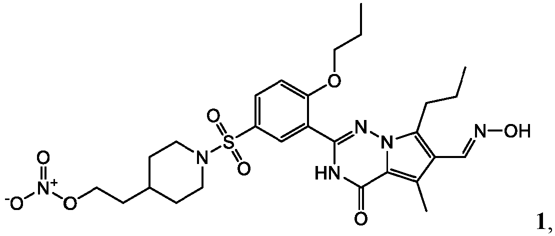 and
wherein said topical composition comprises said Compound **1** in an amount between about 0.7µmol and about 5µmol, preferably in an amount between about 0.8µmol and about 2µmol, and further preferably in an amount of about 1µmol; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous phosphate buffer of a pH of about 6.5 to about 7.5, wherein preferably said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) a thickening agent, wherein said thickening agent is hydroxyethyl cellulose or sodium hyaluronate, preferably wherein said thickening agent is hydroxyethyl cellulose, and wherein the thickening agent is present from about 0.01% (w/w) to about 5% (w/w), preferably about 0.05% (w/w) to about 2% (w/w), more preferably about 0.1% (w/w) to about 1% (w/w), based on the total weight of said topical composition;
   (iii) an antioxidant, wherein said antioxidant is butylated hydroxytoluene, and wherein said antioxidant is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
   (iv) a preservative, wherein said preservative is benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 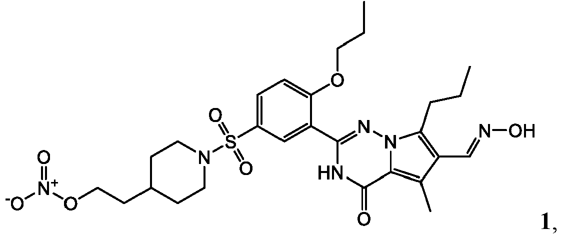 and
wherein said topical composition comprises said Compound **1** in a concentration between about 7µM and about 55µM, preferably in a concentration between about 8µM and about 22µM, further preferably in a concentration between about 10µM and about 12µM, and again further preferably in a concentration of about 11µM; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous phosphate buffer of a pH of about 6.5 to about 7.5, wherein preferably said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) a thickening agent, wherein said thickening agent is hydroxyethyl cellulose or sodium hyaluronate, preferably wherein said thickening agent is hydroxyethyl cellulose, and wherein the thickening agent is present from about 0.01% (w/w) to about 5% (w/w), preferably about 0.05% (w/w) to about 2% (w/w), more preferably about 0.1% (w/w) to about 1% (w/w), based on the total weight of said topical composition;
   (iii) an antioxidant, wherein said antioxidant is butylated hydroxytoluene, and wherein said antioxidant is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
   (iv) a preservative, wherein said preservative is benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 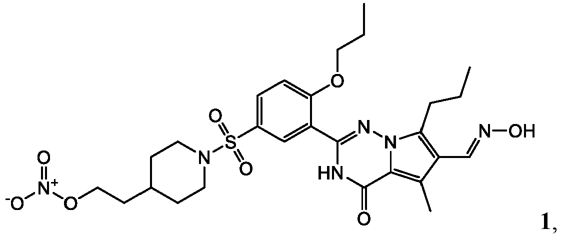 and
wherein said topical composition comprises said Compound **1** in an amount between about 0.7µmol and about 5µmol, preferably in an amount between about 0.8µmol and about 2µmol, and further preferably in an amount of about 1µmol; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous phosphate buffer of a pH of about 6.5 to about 7.5, wherein preferably said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) a thickening agent, wherein said thickening agent is hydroxyethyl cellulose or sodium hyaluronate, preferably wherein said thickening agent is hydroxyethyl cellulose, and wherein the thickening agent is present from about 0.01% (w/w) to about 5% (w/w), preferably about 0.05% (w/w) to about 2% (w/w), more preferably about 0.1% (w/w) to about 1% (w/w), based on the total weight of said topical composition;
   (iii) an antioxidant, wherein said antioxidant is butylated hydroxytoluene, and wherein said antioxidant is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
   (iv) a preservative, wherein said preservative is benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 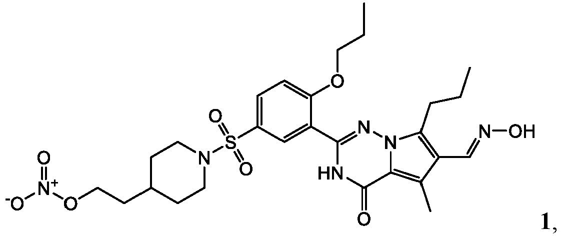 and
wherein said topical composition comprises said Compound **1** in a concentration between about 7µM and about 55µM, preferably in a concentration between about 8µM and about 22µM, and further preferably in a concentration between about 10µM and about 12µM, and again further preferably in a concentration of about 11µM; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous phosphate buffer of a pH of about 6.5 to about 7.5, wherein preferably said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) a thickening agent, wherein said thickening agent is hydroxyethyl cellulose or sodium hyaluronate, preferably wherein said thickening agent is hydroxyethyl cellulose, and wherein the thickening agent is present from about 0.01% (w/w) to about 5% (w/w), preferably about 0.05% (w/w) to about 2% (w/w), more preferably about 0.1% (w/w) to about 1% (w/w), based on the total weight of said topical composition;
   (iii) an antioxidant, wherein said antioxidant is butylated hydroxytoluene, and wherein said antioxidant is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
   (iv) a preservative, wherein said preservative is benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 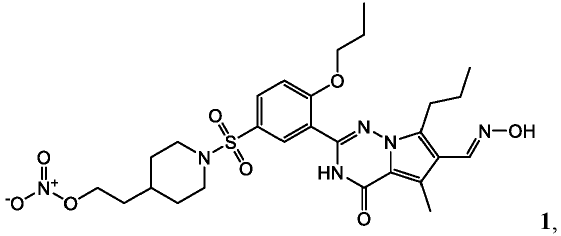 and
wherein said topical composition comprises said Compound **1** in an amount between about 0.7µmol and about 5µmol, preferably in an amount between about 0.8µmol and about 2µmol, and further preferably in an amount of about 1µmol; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous phosphate buffer of a pH of about 6.5 to about 7.5, wherein preferably said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) hydroxyethyl cellulose or sodium hyaluronate, preferably hydroxyethyl cellulose, and wherein hydroxyethyl cellulose or sodium hyaluronate is present from about 0.01% (w/w) to about 5% (w/w), preferably about 0.05% (w/w) to about 2% (w/w), more preferably about 0.1% (w/w) to about 1% (w/w), based on the total weight of said topical composition.
   (iii) butylated hydroxytoluene, and wherein butylated hydroxytoluene is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition
   (iv) benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 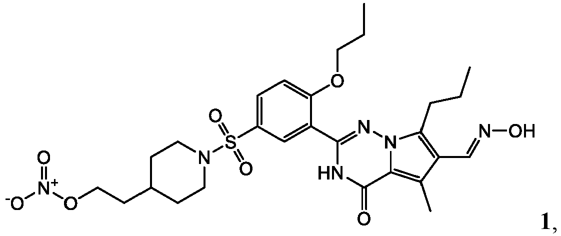 and
wherein said topical composition comprises said Compound **1** in a concentration between about 7µM and about 55µM, preferably in a concentration between about 8µM and about 22µM, further preferably in a concentration between about 10µM and about 12µM, and again further preferably in a concentration of about 11µM; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous phosphate buffer of a pH of about 6.5 to about 7.5, wherein preferably said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) hydroxyethyl cellulose or sodium hyaluronate, preferably hydroxyethyl cellulose, and wherein hydroxyethyl cellulose or sodium hyaluronate is present from about 0.01% (w/w) to about 5% (w/w), preferably about 0.05% (w/w) to about 2% (w/w), more preferably about 0.1% (w/w) to about 1% (w/w), based on the total weight of said topical composition.
   (iii) butylated hydroxytoluene, and wherein butylated hydroxytoluene is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition
   (iv) benzyl alcohol, and wherein said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

In a preferred embodiment, said compound of formula **I** is Compound **1,** 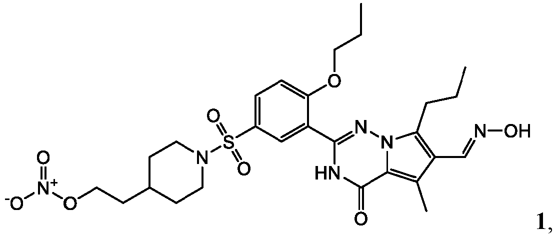 and
wherein said topical composition comprises said Compound **1** in an amount between about 0.8µmol and about 2µmol, preferably in an amount of about 1µmol; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous phosphate buffer of a pH of about 6.5 to about 7.5, wherein preferably said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) hydroxyethyl cellulose, wherein hydroxyethyl cellulose is present from about 0.05% (w/w) to about 2% (w/w), preferably about 0.1% (w/w) to about 1% (w/w), based on the total weight of said topical composition.
   (iii) butylated hydroxytoluene, wherein butylated hydroxytoluene is present from about 0.001% (w/w) to about 0.01% (w/w), preferably about 0.002% (w/w) to about 0.008% (w/w), based on the total weight of said topical composition
   (iv) benzyl alcohol, wherein said benzyl alcohol is present from about 0.1% (w/w) to about 5% (w/w), preferably about 1% (w/w) to about 3% (w/w) and even more preferably of about 2% (w/w) based on the total weight of said topical composition.

In a preferred embodiment, said compound of formula **I** is Compound **1,** 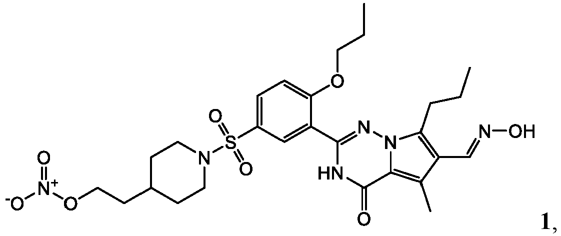 and
wherein said topical composition comprises said Compound **1** in a concentration between about 8µM and about 22µM, preferably in a concentration between about 10µM and about 12µM, and further preferably in a concentration of about 11µM; and
wherein said topical composition is in the form of an aqueous gel, and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
   (i) an aqueous phosphate buffer of a pH of about 6.5 to about 7.5, wherein preferably said aqueous phosphate buffer has a molarity of about 10 to about 15 mM, preferably of about 12mM, and wherein said aqueous phosphate buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
   (ii) hydroxyethyl cellulose, wherein hydroxyethyl cellulose is present from about 0.05% (w/w) to about 2% (w/w), preferably about 0.1% (w/w) to about 1% (w/w), based on the total weight of said topical composition.
   (iii) butylated hydroxytoluene, wherein butylated hydroxytoluene is present from about 0.001% (w/w) to about 0.01% (w/w), preferably about 0.002% (w/w) to about 0.008% (w/w), based on the total weight of said topical composition
   (iv) benzyl alcohol, wherein said benzyl alcohol is present from about 0.1% (w/w) to about 5% (w/w), preferably about 1% (w/w) to about 3% (w/w) and even more preferably of about 2% (w/w) based on the total weight of said topical composition.

Surprisingly, the inventors were able to provide inventive topical compositions, in particular inventive topical compositions comprising Compound **1,** despite the shown de-facto non-solubility of the inventive compounds of formula **I,** in particular of Compound **1,** in physiological aqueous phosphate buffer (pH 7.5). Moreover, the inventive topical compositions showed efficacy and improved wound healing in uPAR deficient mice, a disease-relevant animal model of systemic sclerosis having cutaneous ulcers, at surprisingly very low concentrations of the inventive compounds of formula **I**, in particular of Compound **1.**

As shown in Example 3, Compound **1** shows the profile of an insoluble drug with a determined solubility of 0.0 mg/ml at physiological aqueous phosphate buffer (pH 7.5) and, further, a very pronounced hydrophobicity of calculated logP (clogP) value of 3.79 (See Faller B and Ertl P, Computational approaches to determine drug solubility, Adv Drug Deliv Rev. (2007) 59(7):533-545). Water solubility is an important molecular property for successful drug development as it is a key factor governing drug access to biological membranes. Typically, very poor or no aqueous solubility cannot be compensated by standard formulation developments, in particular due to the unpredictability of the solubility behavior of the very poor or not soluble drug. Poor aqueous solubility is caused by two main factors: i) high lipophilicity and ii) strong intermolecular interactions, which make the solubilization of the solid energetically costly.

As shown further in Example 3, a solubility screening of Compound 1 surprisingly revealed the significant superiority of PEG400, and hereby in particular at very high concentrations of PEG400. Further surprisingly, the use of surfactants for solubilization such as polysorbate 80 and poloxamer 407, typically good solubilization enhancers for lipophilic drugs, showed an 'antagonistic effect" in sense that at 1%, both surfactants lead to an increase in solubility but the amount of drug dissolved did not linearly or not at all increase with higher concentrations of surfactants. This is a very uncommon finding.

Furthermore, and as shown in Example 4, the inventive topical compositions did not demonstrate any negative effect on wound healing properties on an open wound in DU Treatment. This is not only true for the inventive compounds, in particular Compound **1,** but as well for the entirety of applied excipients used in combination herewith. This is additionally remarkable since said preferred topical compositions solely comprises excipients approved for topical treatment, in particular for human use, by the relevant authorities. In addition, said usage of regulatorily approved excipients was also in accordance with the accepted defined concentrations. Thus, the inventors were able to provide efficacious topical compositions solely using admissible and allowed excipients for human use within the accepted concentrations.

The topical composition of the present invention can be used for the treatment of diseases or disorders of the skin being mediated by or resulting from PDE5 activity and/or NO related endothelial dysfunction, in particular, such as ischemic skin ulcers, such as digital ulcers (DU) in systemic sclerosis, preferably for wound healing, further preferably for chronic wound healing, diabetic foot ulcer, leg ulcer, ischemic arterial ulcers, livedoid vasculopathy, Martorell hypertensive ischemic leg ulcer, thromboangiititis obliteragans (Buerger's disease), sickle cell leg ulcer.

Thus, in a further aspect, the present invention provides the topical composition of the present invention for use in a method of topically treating a disease or disorder mediated by PDE5 activity and/or NO related endothelial dysfunction, preferably of a disease or disorder of the skin mediated by PDE5 activity and/or NO related endothelial dysfunction, in a subject, preferably in a human. Preferably, said disease or disorder is selected from ischemic skin ulcers, digital ulcers (DU) in systemic sclerosis, diabetic foot ulcer, leg ulcer, ischemic arterial ulcers, livedoid vasculopathy, Martorell hypertensive ischemic leg ulcer, thromboangiititis obliteragans (Buerger's disease), sickle cell leg ulcer, all of the foregoing preferably for wound healing, further preferably for chronic wound healing. Very preferred, said disease or disorder is systemic sclerosis, and wherein preferably said disease or disorder is digital ulcers in systemic sclerosis.

In another aspect, the present invention provides the topical composition of the present invention for use in a method of topically treating a disease or disorder in a subject, preferably in a human, wherein said disease or disorder is selected from ischemic skin ulcers, digital ulcers (DU) in systemic sclerosis, diabetic foot ulcer, leg ulcer, ischemic arterial ulcers, livedoid vasculopathy, Martorell hypertensive ischemic leg ulcer, thromboangiititis obliteragans (Buerger's disease), sickle cell leg ulcer, all of the foregoing preferably for wound healing, further preferably for chronic wound healing, and wherein preferably said disease or disorder is systemic sclerosis, and again further preferably said disease or disorder is digital ulcers in systemic sclerosis.

In a further aspect, the invention provides a kit comprising:
(i) a first kit component comprising the topical composition of the present invention; and
(ii) a second kit component comprising a patch, a tape, a dressing, a sheet or a device suitable to maintain contact between said topical composition and the skin of a subject, preferably the skin of a human.

A device suitable to maintain contact between the topical composition of the invention and the skin of the subject include, typically and preferably, a patch including a dressing, a tape, a sheet, an adhesive or non-adhesive patch, or any other form known to those skilled in the art such as microneedles. For example, dressings suitable for the present invention and in particular wound dressings, and hereby preferably for chronic wounds, have been described and reviewed (Dabiri G et al, Advances in Wound Care (2016) 5(1):32-41; Shi C et al, Front. Bioeng. Biotechnol. (2020) 8:182).

Topical dressings are typically designed with the physical placement of a patch with an emphasis on localized delivery. They are designed to be used to alleviate localized conditions in the form of an adhesive or non-adhesive patch. Examples of dressing preparations are hydrogel three-dimensional network of hydrophilic polymers; hydrocolloid hydrogel mixed with synthetic rubber and sticky materials; alginate consisting of polysaccharides derived from brown seaweed; foam consisting of polyurethane or is silicone-based with a semi permeability thermal insulation; films consisting typically of adhesive, porous, and thin transparent polyurethane. The dressing preparation optionally contain the active compound, i.e. the compound of formula **I** of the present invention, which is disposed on at one side of the backing of the patch. The adhesive or non-adhesive patch is flexible enough to comply to the skin and has reduced skin irritation and high stability.

In one embodiment, said second kit component comprises a wound dressing.

Disclosed, but not claimed, is a method of preparing the topical composition of the present invention, wherein said method comprises dissolving a compound of formula I in a solvent, wherein preferably said solvent comprises PEG400, and further preferably wherein said solvent comprises PEG400 and water. In a further preferred embodiment, the amount of said PEG 400 is from about 60% to about 75 % (w/w), preferably from about 65% to about 75 % (w/w), and more preferably from about 68 % to about 72 % (w/w) based on the total weight of the topical composition. In another preferred embodiment, the said compound of formula **I** is Compound **1,** and said topical composition comprises said Compound **1** in an amount between about 0.01µmol and about 50µmol, preferably between about 0.1µmol and about 20µmol, further preferably between about 0.2µmol and about 15µmol, further preferably in an amount between about 0.3µmol and about 12µmol, again further preferably in an amount between about 0.3µmol and about 10µmol, and again further preferably in an amount between about 0.3µmol and about 5µmol. In another preferred embodiment, the said compound of formula **I** is Compound **1,** and said topical composition comprises said Compound **1** in a concentration between about 0.1µM and about 500µM, preferably between about 1µM and about 200µM, further preferably between about 2µM and about 150µM, further preferably in a concentration between about 3µM and about 120µM, again further preferably in a concentration between about 3µM and about 100µM, and again further preferably in a concentration between about 3µM and about 50µM.

### EXAMPLES

Materials: The following materials were used to prepare exemplary compositions described herein, all of said materials are known to the skilled person in the art and are further described in detail in Handbook of Pharmaceutical Excipients published jointly by the American Pharmaceutical Association and The Pharmaceutical Society of Great Britain (eds RC Rowe, PJ Sheskey and ME Quinn; Pharmaceutical Press, London, 2009): Hydroxyethyl cellulose (HEC; Natrosol 250H); Butylated hydroxytoluene (Sigma-Aldrich Chemie GmbH); Benzyl alcohol (AppliChem GmbH); PEG 400 (Kollisolv PEG E 400; Sigma-Aldrich Chemie GmbH); Sodium Hyaluronate (Fidia farmaceutici S.p.A.); Sodium dihydrogen phosphate anhydrate (Acros Organics); Sodium hydroxide (PanReac).

Synthesis of Compounds of formula I: Compounds of formula **I** as well as their syntheses are disclosed in WO 2017/085056. In particular Examples 31, 55, 60, 63, 76 and 87 of WO 2017/085056 contain a detailed description of the preparation of preferred compounds of formula **I**. Specifically Example 31 of WO 2017/085056 discloses the synthesis of the very preferred compound of formula I, 2-[1-(3-{6-[(1E)-(hydroxyimino)methyl]-5-methyl-4-oxo-7-propyl-3H,4H-pyrrolo[2,1-f][1,2,4]triazin-2-yl}-4-propoxybenzenesulfonyl)piperidin-4-yl]ethyl nitrate (Compound 1), used in the following examples.

Analytical HPLC: HPLC-UV was used for the analysis of the exemplary compositions described herein. The content of compound **1** in the compositions was within the predefined range of acceptance.

Equipment: Agilent HP 1100 connected to Waters Empower 3 SR3

Analytical conditions: Column: Waters Acquity XBridge C18 3.5 µm 3.0 x 50 mm, Column temp.: 40°C, Mobile phase A: 0.1% formic acid in water, Mobile phase B: Acetonitrile, Flow: 1.0 mL/min, Injection vol.: 20 µL (single injection), detection: 250 nm wavelength.

| **Gradient Table - Time [min]** | **%A** | **%B** |
|---|---|---|
| Initial | 60.0 | 40.0 |
| 4.00 | 23.0 | 77.0 |
| 4.01 | 5.0 | 95.0 |
| 5.00 | 5.0 | 95.0 |
| 5.01 | 60.0 | 40.0 |

All exemplary compositions were clear gel-like solutions by visual inspection and were stable for at least 2 weeks at RT.

Viscosity measurement: The zero viscosity describes the viscosity of the composition at a plateau, which represents the viscosity at rest. The zero viscosity of the exemplary compositions and composition vehicles for Examples 2A to 2D was in the range of 0.8684 [Pa*s], and for the exemplary compositions described and composition vehicles for Examples 2E and 2F, the zero viscosity was in the range of 3.6784 [Pa*s].

Rheological method: Anton Paar GmbH MCR 102, Software: Rheocompass (Anton Paar GmbH), Cone plate geometry (diameter 50 mm), 5 minutes resting phase. 50 measuring points with varying duration of the measurement point (logarithmic): 100 s start value und 0,1 s end value. Segment duration: 759.862 s.

### EXAMPLE 1

### Preparation of composition vehicles

### A. Preparation of Vehicle Composition A

For 100.0 g of Vehicle Composition A, hydroxyethyl cellulose (HEC; 0.25 g) was added in portions to 12 mM phosphate buffer (pH 6.7) and stored below 8°C for dissolution of the thickener. Butylated hydroxytoluene (0.006 g) and benzyl alcohol (2.0 g) were added to PEG 400 (70.0 g), heated to 50°C and cooled down after dissolution. The PEG 400 mixture was added to the aqueous gel (equilibrated to room temperature at phase merging) and stirred with a spatula until a homogenous gel was obtained.

Preparation of phosphate buffer: 12 mM phosphate buffer (pH 6.7) was composed of 2.83 mM Na₂HPO₄ and 9.17 mM NaH₂PO₄ and obtained by adding 402 mg Na₂HPO₄ (MW 142 g / mol) to 1100 mg NaH₂PO₄ (MW 120 g / mol) in demineralized, sterile water to a final volume of 1000 ml.

### B. Preparation of Vehicle Composition B

For 100.0 g of Vehicle Composition **B,** sodium hyaluronate (0.5 g) was added in portions to 12 mM phosphate buffer pH 6.7 and stored below 8°C for dissolution of the thickener. Butylated hydroxytoluene (0.006 g) and benzyl alcohol (2.0 g) were added to PEG 400 (70 g), heated to 50°C and cooled down after dissolution. The PEG 400 mixture was added to the aqueous gel (equilibrated to room temperature at phase merging) and stirred with a spatula until a homogenous gel was obtained.

Preparation of phosphate buffer:12 mM phosphate buffer (pH 6.7) was composed of 2.83 mM Na₂HPO₄ and 9.17 mM NaH₂PO₄ and obtained by adding 402 mg Na₂HPO₄ (MW 142 g / mol) to 1100 mg NaH₂PO₄ (MW 120 g / mol) in demineralized, sterile water to a final volume of 1000 ml.

### EXAMPLE 2

### Preparation of inventive compositions

### A. Preparation of Topical Composition A1

For 100.0 g of Topical Composition **A1,** hydroxyethyl cellulose (HEC; 0.25 g) was added in portions to 12 mM phosphate buffer (pH 6.7) and stored below 8°C for dissolution of the thickener. Butylated hydroxytoluene (0.006 g) and benzyl alcohol (2.0 g) were added to PEG 400 (70.0 g), heated to 50°C and cooled down after dissolution. Compound **1** (0.00018 g; 0.3µmol) was dissolved in the PEG 400 mixture (heated to 50°C for 15 minutes and stirred for 2 hours at room temperature). The PEG 400 mixture was added to the aqueous gel (equilibrated to room temperature at phase merging) and stirred with a spatula until a homogenous gel was obtained.

Preparation of phosphate buffer: 12 mM phosphate buffer (pH 6.7) was composed of 2.83 mM Na₂HPO₄ and 9.17 mM NaH₂PO₄ and obtained by adding 402 mg Na₂HPO₄ (MW 142 g / mol) to 1100 mg NaH₂PO₄ (MW 120 g / mol) in demineralized, sterile water to a final volume of 1000 ml. The composition was all prepared under avoidance of light in a room with minimal UV-light exposure using aluminum foil or amber glass ware.

### B. Preparation of Topical Composition A2

For 100.0 g of Topical Composition **A2,** hydroxyethyl cellulose (HEC; 0.25 g) was added in portions to 12 mM phosphate buffer (pH 6.7) and stored below 8°C for dissolution of the thickener. Butylated hydroxytoluene (0.006 g) and benzyl alcohol (2.0 g) were added to PEG 400 (70.0 g), heated to 50°C and cooled down after dissolution. Compound **1** (0.00061 g; 1µmol) was dissolved in the PEG 400 mixture (heated to 50°C for 15 minutes and stirred for 2 hours at room temperature). The PEG 400 mixture was added to the aqueous gel (equilibrated to room temperature at phase merging) and stirred with a spatula until a homogenous gel was obtained.

Preparation of phosphate buffer: 12 mM phosphate buffer (pH 6.7) was composed of 2.83 mM Na₂HPO₄ and 9.17 mM NaH₂PO₄ and obtained by adding 402 mg Na₂HPO₄ (MW 142 g / mol) to 1100 mg NaH₂PO₄ (MW 120 g / mol) in demineralized, sterile water to a final volume of 1000 ml. The composition was all prepared under avoidance of light in a room with minimal UV-light exposure using aluminum foil or amber glass ware.

### C. Preparation of Topical Composition A3

For 100.0 g of Topical Composition **A3,** hydroxyethyl cellulose (HEC; 0.25 g) was added in portions to 12 mM phosphate buffer (pH 6.7) and stored below 8°C for dissolution of the thickener. Butylated hydroxytoluene (0.006 g) and benzyl alcohol (2.0 g) were added to PEG 400 (70.0 g), heated to 50°C and cooled down after dissolution. Compound **1** (0.0018 g; 3µmol) was dissolved in the PEG 400 mixture (heated to 50°C for 15 minutes and stirred for 2 hours at room temperature). The PEG 400 mixture was added to the aqueous gel (equilibrated to room temperature at phase merging) and stirred with a spatula until a homogenous gel was obtained.

Preparation of phosphate buffer: 12 mM phosphate buffer (pH 6.7) was composed of 2.83 mM Na₂HPO₄ and 9.17 mM NaH₂PO₄ and obtained by adding 402 mg Na₂HPO₄ (MW 142 g / mol) to 1100 mg NaH₂PO₄ (MW 120 g / mol) in demineralized, sterile water to a final volume of 1000 ml. The composition was all prepared under avoidance of light in a room with minimal UV-light exposure using aluminum foil or amber glass ware.

### D. Preparation of Topical Composition A4

For 100.0 g of Topical Composition **A4,** hydroxyethyl cellulose (HEC; 0.25 g) was added in portions to 12 mM phosphate buffer (pH 6.7) and stored below 8°C for dissolution of the thickener. Butylated hydroxytoluene (0.006 g) and benzyl alcohol (2.0 g) were added to PEG 400 (70.0 g), heated to 50°C and cooled down after dissolution. Compound **1** (0.0061 g; 10µmol) was dissolved in the PEG 400 mixture (heated to 50°C for 15 minutes and stirred for 2 hours at room temperature). The PEG 400 mixture was added to the aqueous gel (equilibrated to room temperature at phase merging) and stirred with a spatula until a homogenous gel was obtained.

Preparation of phosphate buffer: 12 mM phosphate buffer (pH 6.7) was composed of 2.83 mM Na₂HPO₄ and 9.17 mM NaH₂PO₄ and obtained by adding 402 mg Na₂HPO₄ (MW 142 g / mol) to 1100 mg NaH₂PO₄ (MW 120 g / mol) in demineralized, sterile water to a final volume of 1000 ml. The composition was all prepared under avoidance of light in a room with minimal UV-light exposure using aluminum foil or amber glass ware.

### E. Preparation of Topical Composition B1

For 100.0 g of Topical Composition **B1**, sodium hyaluronate (0.5 g) was added in portions to 12 mM phosphate buffer pH 6.7 and stored below 8°C for dissolution of the thickener. Butylated hydroxytoluene (0.006 g) and benzyl alcohol (2.0 g) were added to PEG 400 (70 g), heated to 50°C and cooled down after dissolution. Compound **1** (0.00061 g; 1µmol) was dissolved in the PEG 400 mixture (heated to 50°C for 15 minutes and stirred for 2 hours at room temperature). The PEG 400 mixture was added to the aqueous gel (equilibrated to room temperature at phase merging) and stirred with a spatula until a homogenous gel was obtained.

Preparation of phosphate buffer:12 mM phosphate buffer (pH 6.7) was composed of 2.83 mM Na₂HPO₄ and 9.17 mM NaH₂PO₄ and obtained by adding 402 mg Na₂HPO₄ (MW 142 g / mol) to 1100 mg NaH₂PO₄ (MW 120 g / mol) in demineralized, sterile water to a final volume of 1000 ml.

### E. Preparation of Topical Composition B2

For 100.0 g of Topical Composition **B2,** sodium hyaluronate (0.5 g) was added in portions to 12 mM phosphate buffer pH 6.7 and stored below 8°C for dissolution of the thickener. Butylated hydroxytoluene (0.006 g) and benzyl alcohol (2.0 g) were added to PEG 400 (70 g), heated to 50°C and cooled down after dissolution. Compound **1** (0.0061 g; 10µmol) was dissolved in the PEG 400 mixture (heated to 50°C for 15 minutes and stirred for 2 hours at room temperature). The PEG 400 mixture was added to the aqueous gel (equilibrated to room temperature at phase merging) and stirred with a spatula until a homogenous gel was obtained.

Preparation of phosphate buffer:12 mM phosphate buffer (pH 6.7) was composed of 2.83 mM Na₂HPO₄ and 9.17 mM NaH₂PO₄ and obtained by adding 402 mg Na₂HPO₄ (MW 142 g / mol) to 1100 mg NaH₂PO₄ (MW 120 g / mol) in demineralized, sterile water to a final volume of 1000 ml.

All prepared compositions were clear and homogeneous low-viscosity gels, wherein Compound 1 was fully dissolved. All the compositions showed shear-thinning behavior which is typical for polymer-based gel compositions. Their consistency allows easy application and spreading on wounds, whilst preventing running off of the composition.

### EXAMPLE 3

### Solubility Studies

### A. Solubility of Compound 1 in water.

Hereto, 500 mg of Compound 1 was weighed into separate 10 mL amber glass vials. 5.0 ml of solvent was added to each vial to give a nominal concentration of approximately 100 mg/mL The samples were stirred for 24 hours under ambient laboratory conditions. After stirring, aliquots of each suspension were filtered through a 1.0 µm PTFE membrane filter, and 0.5 mL of supernatant accurately transferred into separate 250 mL amber volumetric flasks. The flasks were diluted to volume with acetonitrile to give a nominal concentration of 0.2 mg/mL. The concentration of Compound 1 in each of the sample solutions was determined by HPLC. For each solution, the concentration (mg/mL) of Compound 1 was determined using the HPLC system, the observed solubility was back calculated based on the dilution factor.

Preparation of buffers was as follows:
Phosphate buffer (pH 7): 100 mL of 0.1M potassium dihydrogen orthophosphate was mixed with 58.2 mL of 0.1 M sodium hydroxide solution and adjusted with 1M sodium hydroxide solution. The pH of the buffer was 7.00.
Phosphate buffer (pH 7.5): 100 mL of 0.1M potassium dihydrogen orthophosphate was mixed with 58.2 mL of 0.1M sodium hydroxide solution and adjusted with 1M sodium hydroxide solution. The pH of the buffer was 7.51.
Phosphate buffer (pH 8.5): 100 mL of 0.1 M potassium dihydrogen orthophosphate was mixed with 93.4 mL of 0.1 M sodium hydroxide solution and adjusted with 1M sodium hydroxide solution. The pH of the buffer was 8.50.
Analytical HPLC: HPLC-MS was used for the analysis of the solubility of Compound 1 described herein using as Equipment the Agilent HPLC UV System with the following Analytical conditions: Column: Halo C18, 150 mm * 4.6 mm, 2.7µm. Column temp.: 40°C, mobile phase A: Water/acetonitrile/trifluoro acetic acid (95/5/0.1:v/v/v), mobile phase B: Water/acetonitrile/ trifluoro acetic acid (5/95/0.05:v/v/v), Injection vol.: 5 µL, detection: 250 nm wavelength.

**Table 1. Gradient of mobile phases**

| **Time [min]** | **% of A** | **% of B** |
|---|---|---|
| 0.0 | 100 | 0 |
| 2.00 | 100 | 0 |
| 27.00 | 0 | 100 |
| 30.00 | 0 | 100 |
| 30.1 | 100 | 0 |

**Table 2. Solubility for Compound 1 in various aqueous buffers.**

| **Solvent** | **Determined Solubility (mg/mL)** |
|---|---|
| Aqueous phosphate buffer (pH 7) | 0.0 |
| Aqueous phosphate buffer (pH 7.5) | 0.0 |
| Aqueous phosphate buffer (pH 8.5) | 0.0 |

Compound 1 shows the profile of an insoluble drug with a determined solubility of 0.0 mg/ml at physiological aqueous phosphate buffer (pH 7.5), and further a very pronounced hydrophobicity of calculated logP (clogP) value of 3.79 (See Faller B and Ertl P, Computational approaches to determine drug solubility, Adv Drug Deliv Rev. (2007) 59(7):533-545).

### B. Solubility Screening of Compound 1 in various solvents/solvent systems.

A solubility screening of Compound **1** was effected. Table 3 shows the determined solubility of Compound **1** in the listed solvents or solvent systems. In general, three potential approaches where covered: i) polar organic co-solvents, ii) solubilization by surfactants and iii) oils which may, in particular, serve as a basis of an emulsion gel. A phosphate buffer of pH 6.5 with low molarity was used as this salt and pH is suitable for application on wounds. It is of note that the resulting pH of the screened systems is about 7.3.

**Table 3. Solubility Screening for Compound 1**

| **Solvent/Solvent Systems** | **Determined Solubility (mg/mL)** |
|---|---|
| PEG 400 / PB (30:70, vol:vol) | 0.001 |
| PEG 400 / PB (70:30, vol:vol) | 0.110 |
| 1% polysorbate 80 in PB | 0.077 |
| 3% polysorbate 80 in PB | 0.139 |
| 8% polysorbate 80 in PB | 0.067 |
| 1% poloxamer 407 in PB | 0.270 |
| 10% poloxamer 407 in PB | 0.0232 |
| Medium chain triglycerides (MCT) | 0.282 |
| Isopropyl myristate (IPM) | 0.093 |
| PEG 400 / PB (30:70, vol:vol) + 3% polysorbate 80 | 0.020 |
| PEG 400 / PB (30:70, vol:vol) + 10% poloxamer 407 | 0.054 |
| PEG 400 / PB (30:70, vol:vol) + 3% benzyl alcohol | 0.008 |
| 8% polysorbate 80 in PB + 10% ethanol | 0.043 |
| 8% polysorbate 80 in PB + 3% benzyl alcohol | 0.056 |
| 8% polysorbate 80 in PB + 10% poloxamer 407 | 0.063 |
| 8% polysorbate 80 in PB + 10% MCT | 0.037 |
| Octyldodecanol (Kollicream OD) | 0.028 |
| Polyethylene glycol PEG 400 | 10.5 |

| | |
|---|---|
| PB = Phosphate buffer 10 mmol/L pH 6.5 | |

As confirmed, Compound **1** shows the profile of an insoluble or very poorly soluble drug with pronounced hydrophobicity. Surprisingly, solely PEG400 led to acceptable solubility data. The use of surfactants for solubilization such as polysorbate 80 and poloxamer 407 were used due to its typically good solubilization potential for lipophilic drugs. At 1%, both surfactants lead to an increase in solubility highlighting the potential of this approach. However, surprisingly, amount of drug dissolved did not linearly or not at all increase with higher concentrations of surfactants. This is a very uncommon finding. As opposed to co-solvents, which typically show sigmoid relationships between solvent concentrations and solubility, surfactants typically show a linear relationship in the concentration range where they form micelles. The observed phenomenon of the lack of solubility increase with surfactant concentration is also referred to as the apparent 'antagonistic effect" of different types of cosolvents and/or surfactants. This is, as indicated a very uncommon finding and very rarely seen.

### C. Solubility Studies of Compound 1 in solvent systems comprising PEG400.

A solubility study of Compound 1 in solvent systems comprising PEG400 in various concentrations was effected. Table 4 shows the determined solubility of Compound 1 with various solvent systems comprising PEG400.

**Table 4. Solubility of Compound 1 in solvent systems comprising PEG400.**

| **Solvent/Solvent Systems (vol:vol)** | **Determined Solubility (mg/mL)** |
|---|---|
| PEG400:benzyl alcohol (99:1) | 12693 |
| PEG400:benzyl alcohol:phosphate buffer (89:1:10) | 1768 |
| PEG400:benzyl alcohol:phosphate buffer (79:1:20) | 882 |
| PEG400:benzyl alcohol:phosphate buffer (69:1:30) | 112 |
| PEG400:benzyl alcohol:phosphate buffer (64:1:35) | 37.0 |
| PEG400:benzyl alcohol:phosphate buffer (59:1:40) | 12.8 |
| PEG400:benzyl alcohol:phosphate buffer (54:1:45) | 5.00 |
| PEG400:benzyl alcohol:phosphate buffer (49:1:50) | 2.18 |
| PEG400:benzyl alcohol:phosphate buffer (44:1:55) | 0.861 |

| | |
|---|---|
| Phosphate buffer 12 mmol/L pH 6.7 | |

The results demonstrates a very sharp increase of solubility of Compound 1 upon higher concentrations of PEG400. The resulting very steep solubility curve is depicted in FIG. 3.

### EXAMPLE 4

### Topical, on wound administration of inventive compounds and its effects on healing of full thickness excisional cutaneous wounds of uPAR-/- mice in vivo

The urokinase-type plasminogen activator receptor deficient (uPAR -/-) mouse that reproduces cardinal features of SSc (dermal) vasculopathy and fibrosis is an animal model of systemic sclerosis that is considered as disease-relevant. The uPAR -/- mouse integrates apoptosis of dermal endothelial cells, a decreased density of dermal microvessels and endothelial to mesenchymal transition reflecting vasculopathy with dermal thickening, collagen accumulation, enhanced myofibroblast counts reflecting skin fibrosis. uPAR-/- induces and develops dermal and pulmonary fibrosis (Manetti M et al. Ann Rheum Dis. (2014) 73:1700-1709; Manetti M et al Ann Rheum Dis. (2017) 76:924-934; Schniering J et al., (2019) In Atlas of Ulcers in Systemic Sclerosis. Diagnosis and Management. Matucci-Cerinic M and Denton CP (eds). Springer, Cham, Switzerland, pp. 27-37).

uPAR -/- mice were bred from mating of heterozygotes (uPAR +/-; obtained from Institute for Translational Neurology and Neurology Clinics, University of Minster, Germany). Pups were genotyped and uPAR -/- mice obtained at Mendelian frequency. Two full thickness, excisional cutaneous wounds (6 mm diameter corresponding to an area of 28.3 mm²) were generated, symmetrically to the dorsum midline. To this end, at the day before surgery, mice were anaesthetized with Isoflurane and their dorsum was shaved using Veet^{®} depilatory cream and clippers. At the next following day, preoperative analgesia was administered at 30 min before surgery consisting of 0.1 mg / kg of Buprenorphine and 5 mg / kg of Ketoprofen. Animals were anesthetized with Isoflurane. Two full thickness wounds (including epidermis, dermis, hypodermis, panniculus carnosus; depth about 500 µm) of 6 mm diameter each were created using a biopsy punch and scissors each with identical, at least 10 mm distance left or right from the dorsum midline. To minimize wound contraction, wounds were splinted using a silicon splint (Grace Bio-Labs silicone wound splints, red with suture sites, outer diameter × inner diameter × thickness 14 mm × 7 mm × 0.5 mm). Splints were kept in place using six interrupted surgical sutures (6/0 polyamide-Ethylon) and Superglue (ethyl 2-cyanoacrylate glue) (Park SA et al. Wound Repair Regen. (2014) 22(3):368-380; Park SA et al. Wound Repair Regen. (2015) 23(2):251-261).

The cutaneous ulcers and the splint were protected using a plastic cover maintained using a non-woven adhesive dressing (Hypafix^{®} (Smith & Nephew)) and a semiocclusive adhesive transparent dressing (Tegaderm^{™}, (3M^{™})). Ketoprofen (5 mg / kg) and Buprenorphine (0.05 mg / kg) were administered for 3 days and then Buprenorphine (0.05 mg / kg) as needed. The estimated volume of a cutaneous ulcer was 28.3 mm² area x 0.5 mm depth = 14.15 mm³ or 14.15 µl.

Based on this calculation a dose volume of 25 µl per wound when the wound area was > 50 % of the original size that was reduced to 12.5 µl from the day the wound size was ≤ 50 % less of the original size was appropriate. Given a mean body weight of about 20 g per mouse the dose per body weight was initially 0.75 µg/kg of Compound **1** (in form of the Topical Composition **A2)** as well as Vehicle Composition **A** followed by 0.37 µg/kg of Compound **1** (in form of the Topical Composition **A2)** as well as Vehicle Composition **A** when the wound size was at least halved.

The inventive composition and composition vehicle were administered once daily with identical treatment to both wounds of an individual mouse under a short isoflurane general anaesthesia. To this end, following removal of the dressings, the ulcers were cleaned using saline solution and fibrin tissue or eschar removed. The inventive composition and composition vehicle were added by soft pipetting and the wounds were immediately closed using the dressings as described before.

Groups were (i) uPAR +/+ (wild type) mice with Vehicle Composition **A**, (ii) uPAR -/mice with Vehicle Composition **A**, (iii) uPAR -/- mice with Topical Composition **A2.**

Treatment started at the day of surgery defined as day 0 and was continued once daily until wound closure at day 12-14. In order to record potential treatment effects wounds were photographed daily using a digital camera (Sony 6400) fixed on a Stereo-Microscope. Three high resolution pictures were taken per wound per day. The wound area was measured off-line from the recorded high-resolution photographs by Image J software. Image J analyses of each ulcer were analysed at least twice. Per wound and day of observation after surgery a total of four to six Image J analyses for one ulcer area were done.

The mean of the four to six values was included in the final summary analyses and used for the calculation of % *Wound closure* according to the equation below (Park SA et al. Wound Repair Regen. (2014) 22(3):368-380): $% Wound Closure = \frac{{UA}_{0} - {UA}_{k}}{{UA}_{0}} ∗ 100 %$ wherein UA₀ is ulcer area at observation day 0, UAₖ is ulcer area at observation day k (0≤k≤14).

Following repeated dosing, once daily, on wound administration both Topical Composition **A2** as well as Vehicle Composition **A** were safe and well tolerated. No treatment emergent local or systemic adverse effects were observed over the 12-14 days treatment period and in terms of body weight changes compared to day 0 there were no significant differences between the groups of Topical Composition **A2** and Vehicle Composition **A** up to and including day 8.

Results of the primary efficacy analysis for wound closure are summarized in FIG. 1A and FIG. 1B and Table 5 showing a delayed healing of cutaneous wounds in uPAR -/- mice and effects of topical, on wound administered Topical Composition **A2.**

The calculated % Wound Closure was less in uPAR -/- mice compared to uPAR +/+ wild type mice, both treated once daily, *on wound* with Vehicle Composition **A**. This difference in % Wound Closure between uPAR -/- and uPAR +/+ mice was significant at observation day 3 to day 8. Wound healing was delayed. For example, about 46% wound closure was observed at day 5 for uPAR +/+ mice but at day 7 for uPAR -/- mice. About 60% wound closure in uPAR +/+ mice was found at day 6 versus day 8 in uPAR -/- mice.

Following repeated dosing, once daily, topical, *on wound* administration of Topical Composition_**A2** (in a dose volume of initially 25 µl and then 12.5 µl from the day the wound size was ≤ 50 % less of the original size) to uPAR -/- mice % Wound Closure was significantly improved from observation day 5 through day 8, when compared to Vehicle Composition **A.** Based on the means of % Wound Closure from day 5 to day 8, wound closure appeared accelerated by about 1 day with Compound **1** versus Vehicle (Table 5).

**Table 5. Delayed healing of cutaneous wounds in uPAR -/- mice and effects of topical, on wound administered Topical Composition A2.**

| **% WOUND CLOSURE** | | | | | | | | | |
|---|---|---|---|---|---|---|---|---|---|
| **Mice** | **uPAR +/+ (Wild Type)** | | | **uPAR -/-** | | | | | |
| **Treatment** | **Vehicle (N=11; mice)** | | | **Vehicle (N=7; mice)** | | | **Compound 1 (A2) 1 µM (N=6; mice)** | | |
| **Day** | **Mean** | SEM | N Wounds | **Mean** | SEM | N Wounds | **Mean** | SEM | N Wounds |
| **0** | **0.00** | 0.00 | 11 | **0.00** | 0.00 | 12 | **0.00** | 0.00 | 12 |
| **1** | **-2.50** | 3.71 | 11 | **-0.66** | 1.64 | 12 | **2.17** | 1.76 | 12 |
| **2** | **6.83** | 3.11 | 11 | **3.34** | 1.51 | 12 | **5.82** | 2.11 | 12 |
| **3** | **20.17** | 3.72 | 11 | **6.78** | 2.08 | 12 | **12.61** | 1.86 | 12 |
| **4** | **31.10** | 3.63 | 9 | **12.88** | 1.96 | 12 | **18.11** | 2.99 | 12 |
| **5** | **46.40** | 3.81 | 7 | **21.47** | 2.61 | 12 | **34.35** | 3.76 | 12 |
| **6** | **60.91** | 4.00 | 7 | **31.93** | 3.45 | 11 | **49.71** | 3.39 | 12 |
| **7** | **75.36** | 5.59 | 6 | **46.90** | 3.12 | 11 | **63.55** | 5.31 | 12 |
| **8** | **84.12** | 3.78 | 5 | **61.01** | 5.16 | 8 | **74.76** | 4.89 | 12 |
| **9** | **88.52** | 6.12 | 3 | **78.45** | 5.16 | 5 | **86.95** | 4.61 | 11 |
| **10** | **93.76** | 2.85 | 3 | **87.01** | 2.88 | 4 | **92.91** | 2.79 | 10 |
| **11** | | | | **97.77** | 1.44 | 2 | **95.56** | 2.41 | 8 |
| **12** | | | | **99.49** | 0.51 | 2 | **97.84** | 1.79 | 5 |
| **13** | | | | **100.00** | 0.00 | 1 | **99.94** | 0.06 | 2 |

Tabulated data from Fig 1. For legend see description of FIG. 1. The means ± SEM are shown. Only wounds with an intact silicone ring and stitches, used to avoid wound contraction, were included in the analysis that explains less evaluable wounds over time (Table 5). In the vehicle groups, some mice received vehicle on one wound and sham at the other wound which explains that the number of wounds may be less than the 2-fold of the number of mice enrolled.

Finally, based on the data shown in FIG. 1 and Table 5, the area under the effect curves (AUEC) for % wound closure were calculated.

**Table 6. Delayed healing of cutaneous wounds in uPAR -/- mice and effects of topical, on wound administered Topical Composition A2, AUEC.**

| Mice | Treatment | AUEC (d0-7) | | | Statistics: Post-hoc test with Bonferroni correction | |
|---|---|---|---|---|---|---|
| | | Mean | SD | N | | |
| uPAR+/+ | HEC/PEG Vehicle | 215.77 | 48.23 | 6 | | p=0.0018 |
| uPAR-/- | HEC/PEG Vehicle | 99.83 | 43.35 | 11 | p=0.032 | |
| uPAR-/- | 1µM Compound **1(A2)** | 154.54 | 51.1 | 12 | | |

AUEC were computed (GraphPad Prism 9.0) based on Data shown in Table 5, FIG. 1. Briefly, AUEC between day 0 and 7 were calculated for each wound using R-Studio (AUEC calculations were limited to day 0-7 to assure that individual AUEC curves from at least 6 wounds per group are included in the analysis). Assumptions of normality were assessed using QQplots and Shapiro test by groups (p. values 0.1933; 0.3633; 0.07 for uPAR -/-, Topical Composition **A2;** uPAR -/-, Vehicle Composition **A;** uPAR +/+, Vehicle Composition **A,** respectively) and assumption of homogeneity of variance was performed using Levene test (p. value = 0.702). ANOVA was statistically significant F(2.26) = 11.765, p<0.001. Post-hoc test were corrected using Bonferoni correction (n=3). P values shown in Table 6 for comparisons (i) between uPAR +/+, vehicle and uPAR -/-, vehicle and (ii) between uPAR -/-, vehicle and uPAR -/-, Topical Composition **A2** are from post-hoc tests with Bonferroni correction.

The AUEC for % wound closure was significantly less in uPAR-/- mice compared to uPAR+/+ mice, in line with delayed wound healing in the uPAR deficient mice. Second, compared to vehicle, Compound **1** resulted in a significant increase in AUEC for % wound closure in uPAR -/- mice confirming improved wound healing caused by Compound **1.** Numerically, about half of the wound closure delay associated with uPAR deficiency was restored by Compound **1** (Table 6).

In order to address the systemic exposure following topical, *on wound* administration of Compound **1,** in a satellite study, plasma concentrations of Compound **1** was measured. To this end, eight male C57BL/6J mice (12 weeks of age, body weight 26.8 ± 0.4 g (Mean ± SD)) underwent surgery for generation of one full thickness, excisional, cutaneous wounds, in an identical procedure as described above. The study was performed in C57BL/6J mice due to limited availability of uPAR -/- mice. As described before, Compound **1** (in form of Topical Composition **A2)** was added in a dose volume of 25 µl to the wounds as a single dose. The dose related to body weight was approximately 0.565 µg / kg (from a total of 25 µl of 1 µM Compound **1** per mouse). Peripheral venous blood was sampled from the tail tip (or by cardiac puncture for the final sampling) in four mice at time points (min) 1, 5, 15, 30, 60 *post* administration of Compound **1** and in another four mice at time points (h) 1, 2, 4, 6, 24 *post* administration of Compound **1.** Plasma was generated and Compound **1** measured by LC-MS/MS. The LLOQ for Compound **1** was at 800 pg / ml corresponding to 1.32 nM. Plasma Compound **1** remained below LLOQ (i.e., < 1.32 nM) at all time points and mice. Considering the plasma protein binding of Compound **1** in mice with unbound fractions (fu) of 0.08 % for Compound **1** the unbound plasma concentrations should have been < 1.06 pM for Compound **1,** corresponding to < 2.12 pM for Compound **1** when extrapolated to administration to two wounds per mice as this was done in the main efficacy study, under the assumption of dose linearity. These values are much less than the IC50 values of Compound **1** (1.18 nM) and the IC40 values for relaxation of rat aortic rings (Compound **1,** IC40 at 8 pM, as observed in earlier effected measurements. Based on these findings, local rather than systemic exposure should explain the improved wound healing with topical, on wound of Compound **1** observed in this study.

In conclusion, in uPAR-/- mice, an accepted disease-relevant animal model of SSc, the healing of full thickness excisional cutaneous wounds, that was delayed compared to uPAR +/+ wild type mice, was improved by once daily, topical administration of Compound **1** in form of the Topical Composition **A2.** Local rather than systemic exposure accounts for the improved wound healing following topical Compound 1 treatment.

### EXAMPLE 5

### Topical administration of inventive compounds and its effects on ulcer core perfusion in full thickness excisional cutaneous wounds of C57Bl/6J mice in vivo

Effects of a single dose of Topical Composition **A2,** Topical Composition **A4,** and Vehicle Composition **A**, when administered on the perfusion of the ulcer core of full thickness excisional cutaneous wounds in C57B1/6J mice was investigated. Thus, Topical Compositions **A2** and **A4** were applied in a dose volume of 25 µl per wound. Each individual mouse had surgery for two wounds located symmetrical to the dorsal midline using 8 mm diameter punch biopsy devices.

Topical Compositions **A2** and **A4** or Vehicle Composition **A** were randomly administered to one or the other wound under a blinded protocol. This procedure enabled an intra-individual comparison between Compound **1** and its composition vehicle. Perfusion of the ulcer core as well as of the periulcer region was separately measured by Laser Speckle Contrast Imaging (LSCI) / Laser Speckle Contrast Analysis (LASCA) using a Perimed device. LASCA wound perfusion measurements of digital ulcers in SSc were previously shown to correlate with time to healing (Barsotti S et al Clin Rheumatol. (2020) 39(1):69-75).

In detail, a single application of Topical Compositions **A2** and **A4** or Vehicle Composition **A,** each at a volume of 25 µl per wound was randomly administered either to the right or left of two full thickness cutaneous wounds per mouse generated by 8 mm punch biopsies and symmetrically to the dorsal midline, at 24 h after surgery. Perfusion was measured by LSCI and recorded as arbitrary perfusion units (PU) before administration of Topical Compositions **A2** and **A4** or Vehicle Composition **A** to determine perfusion at baseline (PU₀) and at time points (t) from 6 min to 33 min after administration in 3 min intervals followed by measurements at 60, 120 and 240 min (PUt with 6≤t≤240).

Results from two types of data analyses are shown in FIG. 2A and FIG. 2B. All compositions applied were well tolerated. Local or systemic adverse events were not reported. In all analyses there was a trend for a superior ulcer core perfusion with Compound **1** compositions, i.e. Topical Compositions **A2** and **A4** compared to Vehicle Composition **A** at almost all times of measurements. Significance versus t₀ was found for both Compound **1** compositions tested at 60 and 30 min, respectively in the ΔΔΔPU(Cpd **1**_{corr}-VEH_{corr}) analysis but not in any other analyses including calculations of area under the effect curve or from the periulcer area. Taken together, these observations demonstrate that topical, *on wound* administration of Compound **1** formulated in Vehicle Composition **A** increases perfusion of ulcer core from (acute) full thickness excisional cutaneous wounds in healthy C57Bl/6J mice.

### EXAMPLE 6

### 7-day dermal wound GLP toxicity study in Göttingen minipigs with a 7-day recovery using inventive topical compositions

In a 7-day dermal wound GLP toxicity study with a 7-day recovery, Göttingen minipigs are treated with Compound **1** formulated in Vehicle Composition **A** at concentrations of 6µM, 24µM and 96µM, and Vehicle Composition **A** alone as control. Hereto, the inventive topical composition and vehicle composition are dermally, topically applied once daily for 7 days followed by a 7-day recovery period to a created wound site and to an intact skin site (8 treatment sites).

Anesthesia/Tranquilization/Analgesics. The animals are pre-medicated with analgesics prior to wound creation. The animals are sedated with an injectable anesthetic and may also be supplemented with isoflurane administered via face mask if necessary. Following surgery an Anesthesia Reversal may be administered. Analgesics are administered up to 5 times following the day of wound creation and on an as needed basis during the treatment period. Analgesics may include an opioid and/or a NSAID. An appropriate antibiotic to reduce the potential of infection are administered before wound creation on Day 1, and again on Days 3 and 5. Prior to the start of treatment a full thickness wound will be created on each animal (wound type = 1 cm punch biopsy). Following dermal application, the wound site are covered with a bandage. Bandages are changed at the time of dose administration.

Evaluations and Measurements. Mortality/morbidity (twice daily); cage-side observations (daily); detailed observations (weekly starting 1 week prior to dosing); draize dermal scoring (daily); body weight (weekly); food consumption (daily, qualitative). Wound evaluations (prior to each dose and at termination). Wound areas are assessed at each dressing change for the presence of inflammation, granulation tissue, wound measurements, condition of skin surrounding the wound, and/or presence of exudate. Inflammation are scored according to modified draize scoring criteria. Photography (prior to each dose and at termination). A digital photo is taken of each study animal's test sites, individually. The study number, animal number, test site and study interval relevant to each photo appears on a cage card or label within the field of the photo along with the date. The digital camera is positioned in such a manner that all animals are photographed from the same distance, same orientation, at the same resolution and with a millimeter ruler in the photograph to provide a gauge for magnification of the image, using a stand to hold the camera. The distance from the camera lens to the punch hole site in each minipig is documented.

Toxicokinetic Sample Collection. Days 1 and 7: Assessment of toxicokinetic parameters such as Cmax, tmax, AUC, dose proportionality for a single analyte and 2 metabolites and single dose route. Samples will be taken from all animals at 6 time points.

Assessments. Day 8 (main) or Day 15 (recovery). Macroscopic examination, organ weights and tissue retention. Moreover, histopathology examination (main and recovery) - all animals (standard tissues including treated and untreated skin sites). Morphometry: Measurements for each punch for each minipig are made to determine the approximate size of the punch hole (including measuring the circumference of the open area, longest diameter of the open punch hole and diameter perpendicular to the longest diameter) using standard parameters. In particular, the area of the wound site (in mm²) remaining open at the time of the photograph is determined. The reduction in measured punch hole area is calculated relative to the Day 1 measurements and expressed as percent closed for each site at each time point. Group means are calculated by treatment group and by time point for measured and calculated parameters.

## Claims

1. A topical composition comprising a compound of formula **I** or a pharmaceutically acceptable salt thereof 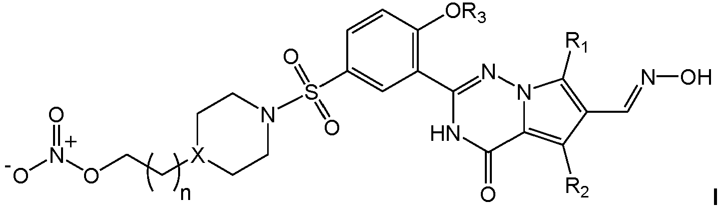 wherein
R₁ is ethyl or propyl, preferably propyl;
R₂ is methyl or ethyl, preferably methyl;
R₃ is ethyl or propyl, preferably propyl;
X is N or CH, preferably CH;
n is 1 or 2, preferably n=1;
wherein said topical composition comprises a solvent; and wherein said solvent comprises PEG 400, and wherein the amount of said PEG 400 is from about 60% to about 75 % (w/w) based on the total weight of the topical composition, wherein the term "about" means ± 10%.

2. The topical composition of claim 1, wherein said compound of formula **I** is Compound 1

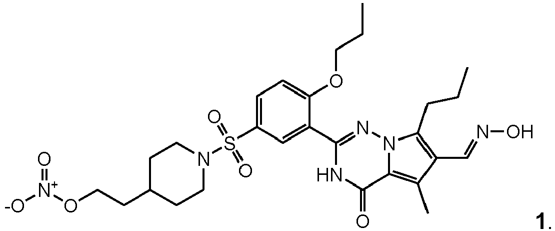

3. The composition of claim 1 or claim 2, wherein said compound of formula **I**, preferably said Compound 1, is present from about 0.000006% (w/w) to about 0.03% (w/w), preferably from about 0.00006% (w/w) to about 0.01% (w/w), more preferably from about 0.0001% (w/w) to about 0.01% (w/w), and again further preferably from about 0.0005% (w/w) to about 0.01% (w/w) based on the total weight of said topical composition.

4. The topical composition of any one of the preceding claims, wherein said topical composition is a liquid topical composition.

5. The topical composition of claim 4, wherein said liquid topical composition is in the form of an aqueous gel.

6. The topical composition of any one of the preceding claims, wherein said topical composition comprises a solvent, wherein said topical composition is in the form of an aqueous gel, and wherein said solvent comprises PEG 400 and water.

7. The topical composition of any one of the preceding claims, wherein the amount of said PEG 400 is from about 65% to about 75 % (w/w), preferably from about 68 % to about 72 % (w/w) based on the total weight of the topical composition.

8. The topical composition of any one of the preceding claims, wherein said topical composition comprises a thickening agent, wherein preferably said thickening agent is selected from the group consisting of carboxymethyl cellulose, hydroxyethyl cellulose, sodium hyaluronate and polyvinylpyrrolidone.

9. The topical composition of any one of the preceding claims, wherein said topical composition comprises an antioxidant, wherein preferably said antioxidant is butylated hydroxytoluene.

10. The topical composition of any one of the preceding claims, wherein said topical composition comprises a preservative, wherein preferably said preservative is benzyl alcohol.

11. The topical composition of any one of the preceding claims, wherein said compound of formula **I** is Compound **1,** 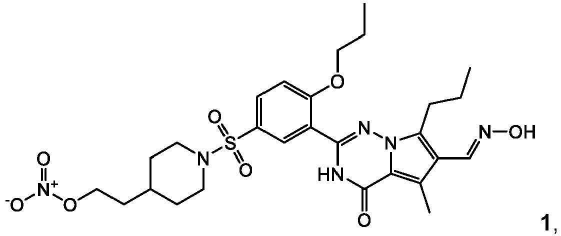 and
wherein said topical composition comprises said Compound 1 in a concentration between about 7µM and about 55µM, preferably in an amount between about 8µM and about 22µM, further preferably in a concentration between about 10µM and about 12µM, and again further preferably in a concentration of about 11µM; and
wherein said topical composition is in the form of an aqueous gel, and wherein said topical composition comprises a solvent; and wherein said solvent is a combination of PEG 400 and water, and wherein the amount of said PEG 400 is from about 60% to about 75 % (w/w), preferably from about 65% to about 75 % (w/w), and more preferably from about 68 % to about 72 % (w/w) based on the total weight of the topical composition;
wherein said topical composition comprises
(i) an aqueous buffer, preferably an aqueous phosphate buffer, of a pH of about 6.5 to about 7.5, wherein preferably said aqueous buffer has a molarity of about 10 to about 15 mM, preferably of about 12mM, and wherein said aqueous buffer is present from about 20% (w/w) to about 30% (w/w), preferably about 22% (w/w) to about 28% (w/w), more preferably about 25% (w/w) to about 28% (w/w) and even more preferably of about 27% (w/w) to about 28% (w/w) based on the total weight of said topical composition;
(ii) hydroxyethyl cellulose or sodium hyaluronate, preferably hydroxyethyl cellulose, and wherein preferably hydroxyethyl cellulose or sodium hyaluronate, preferably hydroxyethyl cellulose, is present from about 0.01% (w/w) to about 5% (w/w), preferably about 0.05% (w/w) to about 2% (w/w), more preferably about 0.1% (w/w) to about 1% (w/w), based on the total weight of said topical composition;
(iii) butylated hydroxytoluene, wherein said butylated hydroxytoluene is present from about 0.001% (w/w) to about 0.1% (w/w), preferably about 0.001% (w/w) to about 0.01% (w/w), more preferably about 0.002% (w/w) to about 0.008% (w/w), based on the total weight of said topical composition; and
(iv) benzyl alcohol, wherein preferably said benzyl alcohol is present from about 0.01% (w/w) to about 5% (w/w), preferably about 0.1% (w/w) to about 5% (w/w), more preferably about 1% (w/w) to about 3% (w/w) and even more preferably of about 2% (w/w) based on the total weight of said topical composition.

12. The topical composition of any one of the claims 1 to 11 for use in a method of topically treating a disease or disorder mediated by PDE5 activity and/or NO related endothelial dysfunction, preferably of a disease or disorder of the skin mediated by PDE5 activity and/or NO related endothelial dysfunction, in a subject, preferably in a human.

13. The topical composition for use according to claim 12, wherein said disease or disorder is selected from ischemic skin ulcers, digital ulcers (DU) in systemic sclerosis, diabetic foot ulcer, leg ulcer, ischemic arterial ulcers, livedoid vasculopathy, Martorell hypertensive ischemic leg ulcer, thromboangiititis obliteragans (Buerger's disease), sickle cell leg ulcer, all of the foregoing preferably for wound healing, further preferably for chronic wound healing.

14. The topical composition for use according to claim 12 or claim 13, wherein said disease or disorder is systemic sclerosis, and wherein preferably said disease or disorder is digital ulcers in systemic sclerosis.

15. A kit comprising:
(i) a first kit component comprising the topical composition of any one of claims 1 to 11; and
(ii) a second kit component comprising a patch, a tape, a dressing, a sheet or a device suitable to maintain contact between said topical composition and the skin of a subject, preferably the skin of a human.

## Patentansprüche

1. Topische Zusammensetzung, die eine Verbindung der Formel **I** oder ein pharmazeutisch verträgliches Salz von dieser enthält 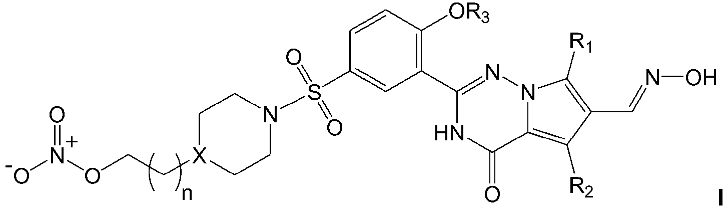 wobei es sich
bei R₁ um ein Ethyl oder Propyl, vorzugsweise Propyl, handelt,
bei R₂ um ein Methyl oder Ethyl, vorzugsweise Methyl, handelt,
bei R₃ um ein Ethyl oder Propyl, vorzugsweise Propyl, handelt,
bei X um N oder CH, vorzugsweise CH, handelt,
n gleich 1 oder 2, vorzugsweise gleich 1, ist,
wobei die topische Zusammensetzung ein Lösungsmittel enthält, und wobei das Lösungsmittel PEG 400 enthält, und wobei die Menge des PEG 400 etwa 60 % bis etwa 75 % (w/w), bezogen auf das Gesamtgewicht der topischen Zusammensetzung, beträgt, wobei der Ausdruck "etwa" ± 10% bedeutet.

2. Topische Zusammensetzung nach Anspruch 1, in der es sich bei der Verbindung nach Formel I um die Zusammensetzung 1 handelt

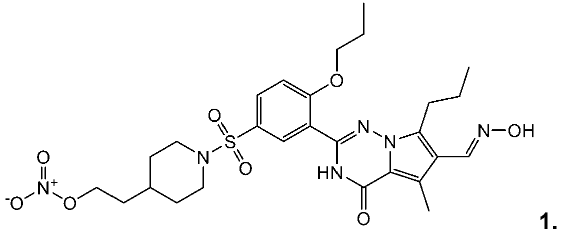

3. Zusammensetzung nach Anspruch 1 oder Anspruch 2, in der der Anteil der Verbindung nach Formel I, vorzugsweise der Verbindung 1, etwa 0,000006 % (w/w) bis etwa 0,03 % (w/w), vorzugsweise etwa 0,00006 % (w/w) bis etwa 0,01 % (w/w), noch vorzugsweiser etwa 0,0001 % (w/w) bis etwa 0,01 % (w/w) und wiederum noch vorzugsweiser etwa 0,0005 % (w/w) bis etwa 0,01% (w/w), beträgt, jeweils bezogen auf das Gesamtgewicht der topischen Zusammensetzung.

4. Topische Zusammensetzung nach einem der vorhergehenden Ansprüche, wobei es sich bei der topischen Zusammensetzung um eine flüssige topische Zusammensetzung handelt.

5. Topische Zusammensetzung nach Anspruch 4, wobei die flüssige topische Zusammensetzung die Form eines wässrigen Gels aufweist.

6. Topische Zusammensetzung nach einem der vorhergehenden Ansprüche, wobei die topische Zusammensetzung ein Lösungsmittel enthält, die Form eines wässrigen Gels aufweist und das Lösungsmittel PEG 400 und Wasser enthält.

7. Topische Zusammensetzung nach einem der vorhergehenden Ansprüche, in der die Menge an PEG 400 etwa 65 % bis etwa 75 % (w/w), vorzugsweise etwa 68 % bis etwa 72 % (w/w), beträgt, jeweils bezogen auf das Gesamtgewicht der topischen Zusammensetzung.

8. Topische Zusammensetzung nach einem der vorstehenden Ansprüche, wobei die topische Zusammensetzung ein Verdickungsmittel enthält und das Verdickungsmittel vorzugsweise aus einer Gruppe bestehend aus Carboxymethylcellulose, Hydroxyethylcellulose, Natriumhyaluronat und Polyvinylpyrrolidon ausgewählt wurde.

9. Topische Zusammensetzung nach einem der vorhergehenden Ansprüche, wobei die topische Zusammensetzung ein Antioxidans umfasst und es sich bei diesem Antioxidans vorzugsweise um butyliertes Hydroxytoluol handelt.

10. Topische Zusammensetzung nach einem der vorhergehenden Ansprüche, wobei die topische Zusammensetzung ein Konservierungsmittel enthält und es sich bei diesem Konservierungsmittel vorzugsweise um Benzylalkohol handelt.

11. Topische Zusammensetzung nach einem der vorstehenden Ansprüche, wobei es sich bei der Verbindung nach Formel **I** um die Verbindung **1** handelt, 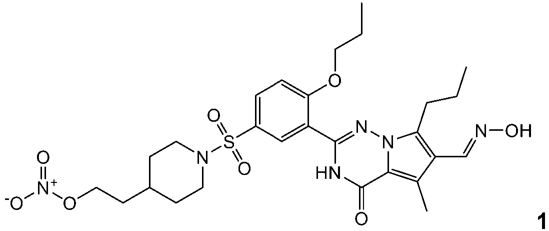 und
wobei die topische Zusammensetzung die Verbindung **1** in einer Konzentration zwischen etwa 7 µM und etwa 55 µM, vorzugsweise in einer Menge zwischen etwa 8 µM und etwa 22 µM, noch vorzugsweise in einer Konzentration zwischen etwa 10 µM und etwa 12 µM, und wiederum weiter vorzugsweise in einer Konzentration von etwa **11** µM enthält; und
wobei die topische Zusammensetzung die Form eines wässrigen Gels aufweist und ein Lösungsmittel enthält, das aus einer Kombination aus PEG 400 und Wasser besteht und in der die Menge an PEG 400 etwa 60 % bis etwa 75 % (w/w), bevorzugt etwa 65 % bis etwa 75 % (w/w), und noch vorzugsweiser etwa 68 % bis etwa 72 % (m/m), beträgt, jeweils bezogen auf das Gesamtgewicht der topischen Zusammensetzung,
wobei die topische Zusammensetzung jeweils die folgenden Bestandteile enthält:
(i) einen wässrigen Puffer, vorzugsweise einen wässrigen Phosphatpuffer, mit einem pH-Wert von etwa 6,5 bis etwa 7,5, wobei der besagte wässriger Puffer bevorzugt eine Molarität von etwa 10 bis etwa 15 mM, vorzugsweise von etwa 12 mM aufweist, und wobei der besagte wässriger Puffer etwa 20 % (w/w) bis etwa 30 % (w/w), vorzugsweise etwa 22 % (w/w) bis etwa 28 % (w/w), noch vorzugsweiser etwa 25 % (w/w) bis etwa 28 % (w/w), und noch vorzugsweiser etwa 27 % (w/w) bis etwa 28 % (w/w) des Gesamtgewichts der besagten topischen Zusammensetzung ausmacht;
(ii) Hydroxyethylcellulose oder Natriumhyaluronat, vorzugsweise Hydroxyethylcellulose, und wobei vorzugsweise Hydroxyethylcellulose oder Natriumhyaluronat, vorzugsweise Hydroxyethylcellulose, etwa 0,01 Gew.-% bis etwa 5 Gew.-%, vorzugsweise etwa 0,05 Gew.-% bis etwa 2 Gew.-%, noch vorzugsweiser etwa 0,1 Gew.-% bis etwa 1 Gew.-%, des Gesamtgewichts der besagten topischen Zusammensetzung ausmacht;
(iii) butyliertes Hydroxytoluol, wobei das butylierte etwa 0,001 Gew.-% bis etwa 0,1 Gew.-%, vorzugsweise etwa 0,001 Gew.-% bis etwa 0,01 Gew.-%, noch vorzugsweise etwa 0,002 Gew.-% bis etwa 0,008 Gew.-%, des Gesamtgewichts der topischen Zusammensetzung ausmacht, und
(iv) Benzylalkohol, wobei vorzugsweise der Benzylalkohol etwa 0,01 % (w/w) bis etwa 5 % (w/w), vorzugsweise etwa 0,1 % (w/w) bis etwa 5 % (w/w), noch vorzugsweiser etwa 1 % (w/w) bis etwa 3 % (w/w), und noch vorzugsweiser etwa 2 % (w/w), des Gesamtgewichts der topischen Zusammensetzung ausmacht.

12. Topische Zusammensetzung nach einem der Ansprüche 1 bis 11 zur Verwendung in einem Verfahren zur topischen Behandlung einer durch PDE5-Aktivität und/oder NO-bezogene endotheliale Dysfunktion vermittelten Krankheit oder Störung, vorzugsweise einer durch PDE5-Aktivität und/oder NO-bezogene endotheliale Dysfunktion vermittelten Krankheit oder Störung der Haut, bei einem Probanden, vorzugsweise einem Menschen.

13. Topische Zusammensetzung zur Verwendung nach Anspruch 12, wobei die Krankheit oder Störung ausgewählt ist aus der Gruppe von ischämischen Hautgeschwüren, digitalen Geschwüren (DU) bei systemischer Sklerose, diabetischem Fußgeschwür, Beingeschwür, ischämischen arteriellen Geschwüren, Livedoid-Vaskulopathie, hypertensivem ischämischen Beingeschwür nach Martorell, Thromboangiitis obliterans (Buerger-Krankheit), Sichelzellen-Beingeschwür, und bei allen der vorgenannten Krankheiten oder Störungen vorzugsweise zur Verwendung zur Wundheilung, noch vorzugsweiser zur Heilung chronischer Wunden.

14. Topische Zusammensetzung zur Verwendung gemäß Anspruch 12 oder Anspruch 13, wobei es sich bei der fraglichen Krankheit oder Störung um systemische Sklerose und vorzugsweise die Krankheit oder Störung digitale Ulzera bei systemischer Sklerose handelt.

15. Ein Set bestehend aus den folgenden Komponenten:
(i) eine erste Komponente, die die topische Zusammensetzung nach einem der Ansprüche 1 bis 11 enthält, und
(ii) eine zweite Komponente, die ein Pflaster, ein Klebeband, einen Verband, ein Tuch oder ein Produkt, das geeignet ist, den Kontakt zwischen der topischen Zusammensetzung und der Haut eines Probanten, vorzugsweise der Haut eines Menschen, aufrechtzuerhalten.

## Revendications

1. Composition topique comprenant un composé de formule **I,** ou un sel pharmaceutiquement acceptable de celui-ci, 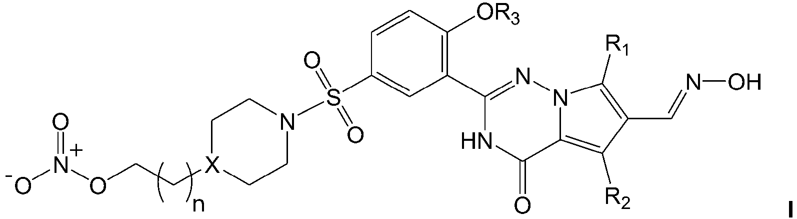 dans laquelle
R₄ représente éthyle ou propyle, de préférence propyle ;
R₂ représente méthyle ou éthyle, de préférence méthyle ;
R₃ représente éthyle ou propyle, de préférence propyle ;
X représente N ou CH, de préférence CH ;
n représente les nombres 1 ou 2, de préférence n=1 ;
ladite composition comprenant un solvant contenant du PEG 400, la quantité dudit PEG 400 représentant environ entre 60 % et 75 % en poids, par rapport au poids total de la composition, où le terme « environ » signifie ± 10%.

2. Composition topique selon la revendication 1, **caractérisée en ce que** ledit composé de formule **I** est le Composé 1.

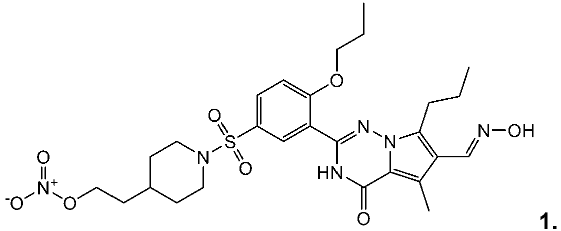

3. Composition selon l'une quelconque des revendications 1 et 2, **caractérisée en ce que** ledit composé de formule **I**, de préférence le Composé 1, est présent à une teneur comprise entre environ 0,000006 % et 0,03 % en poids, préférentiellement entre environ 0,00006 % et 0,01 % en poids, plus préférentiellement entre environ 0,0001 % et 0,01 % en poids, et mieux encore entre environ 0,0005 % et 0,01 % en poids, par rapport au poids total de ladite composition.

4. Composition topique selon l'une quelconque des revendications précédentes, **caractérisée en ce qu'**elle se présente sous forme de liquide.

5. Composition topique selon la revendication 4, **caractérisée en ce que** ladite composition liquide se présente sous forme de gel aqueux.

6. Composition topique selon l'une quelconque des revendications précédentes, **caractérisée en ce qu'**elle comprend un solvant et se présente sous forme de gel aqueux, ledit solvant étant composé de PEG 400 et d'eau.

7. Composition topique selon l'une quelconque des revendications précédentes, **caractérisée en ce que** la proportion dudit PEG 400 est comprise entre environ 65 % et 75 % en poids, préférentiellement entre environ 68 % et 72 % en poids, par rapport au poids total de ladite composition.

8. Composition topique selon l'une quelconque des revendications précédentes, **caractérisée en ce qu'**elle comprend un agent épaississant, ledit agent étant choisi de préférence dans le groupe constitué par la carboxyméthylcellulose, l'hydroxyéthylcellulose, l'hyaluronate de sodium et la polyvinylpyrrolidone.

9. Composition topique selon l'une quelconque des revendications précédentes, **caractérisée en ce qu'**elle comprend un antioxydant, ledit antioxydant étant de préférence du butylhydroxytoluène.

10. Composition topique selon l'une quelconque des revendications précédentes, **caractérisée en ce qu'**elle comprend un conservateur, ledit conservateur étant de préférence de l'alcool benzylique.

11. Composition topique selon l'une quelconque des revendications précédentes, **caractérisée en ce que** ledit composé de formule **I** est le Composé **1** 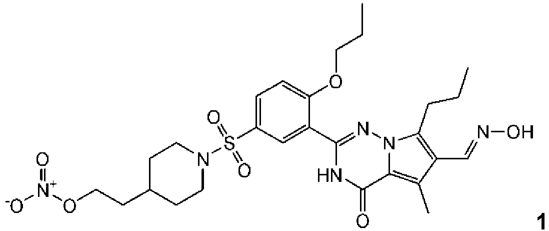
et que ledit Composé **1** est présent dans ladite composition à une concentration comprise entre environ 7 µM et 55 µM, préférentiellement entre environ 8 µM et 22 µM, plus préférentiellement entre environ 10 µM et 12 µM, et mieux encore d'environ 11 µM ;
ladite composition se présente sous forme de gel aqueux et comprend un solvant, ledit solvant étant une combinaison de PEG 400 et d'eau, la quantité dudit PEG 400 représentant environ entre 60 % et 75 % en poids, préférentiellement entre 65 % et 75 % en poids, et plus préférentiellement entre 68 % et 72 % en poids, par rapport au poids total de ladite composition ;
ladite composition comprend :
(i) un tampon aqueux, de préférence un tampon phosphate aqueux, à pH compris entre environ 6,5 et 7,5, ledit tampon ayant une molarité comprise entre environ 10 et 15 mM, de préférence d'environ 12 mM, et étant présent à une teneur comprise entre environ 20 % et 30 % en poids, préférentiellement entre environ 22 % et 28 % en poids, plus préférentiellement entre environ 25 % et 28 % en poids, et mieux encore entre environ 27 % et 28 % en poids, par rapport au poids total de ladite composition ;
(ii) l'hydroxyéthylcellulose ou l'hyaluronate de sodium, de préférence l'hydroxyéthylcellulose, ladite hydroxyéthylcellulose ou ledit hyaluronate de sodium, de préférence l'hydroxyéthylcellulose, étant présent(e) à une teneur comprise entre environ 0,01 % et 5 % en poids, préférentiellement entre environ 0,05 % et 2 % en poids, plus préférentiellement entre environ 0,1 % et 1 % en poids, par rapport au poids total de ladite composition ;
(iii) le butylhydroxytoluène, ledit butylhydroxytoluène étant présent à une teneur comprise entre environ 0,001 % et 0,1 % en poids, préférentiellement entre environ 0,001 % et 0,01 % en poids, plus préférentiellement entre environ 0,002 % et 0,008 % en poids, par rapport au poids total de ladite composition ; et
(iv) l'alcool benzylique, ledit alcool benzylique étant présent à une teneur comprise entre environ 0,01 % et 5 % en poids, préférentiellement entre environ 0,1 % et 5 % en poids, plus préférentiellement entre environ 1 % et 3 % en poids, et mieux encore d'environ 2 % en poids, par rapport au poids total de ladite composition.

12. Composition topique selon l'une quelconque des revendications 1 à 11, **caractérisée en ce qu'**elle est destinée à être utilisée dans un procédé de traitement topique d'une maladie ou d'un trouble médié par l'activité de la PDE5 et/ou un dysfonctionnement endothélial lié au NO, préférentiellement d'une maladie ou d'un trouble de la peau médié par l'activité de la PDE5 et/ou un dysfonctionnement endothélial lié au NO, chez un sujet, de préférence un être humain.

13. Composition topique destinée à être utilisée selon la revendication 12, **caractérisée en ce que** ladite maladie ou ledit trouble est choisi parmi les ulcères cutanés ischémiques, les ulcères digitaux (UD) de la sclérose systémique, l'ulcère du pied diabétique, l'ulcère de la jambe, les ulcères artériels ischémiques, la vasculopathie livédoïde, l'ulcère hypertensif de Martorell, la thromboangéite oblitérante (maladie de Buerger), l'ulcère de jambe drépanocytaire, tous les éléments précités de préférence dans le cadre de la cicatrisation de plaies, plus préférentiellement dans celui de la cicatrisation de plaies chroniques.

14. Composition topique destinée à être utilisée selon l'une quelconque des revendications 12 et 13, **caractérisée en ce que** ladite maladie ou ledit trouble est la sclérose systémique, ladite maladie ou ledit trouble correspondant de préférence aux ulcères digitaux de la sclérose systémique.

15. Kit comprenant :
(i) un premier composant comprenant la composition topique selon l'une quelconque des revendications 1 à 11 ; et
(ii) un second composant comprenant un patch, un ruban, un pansement, une feuille ou un dispositif propre à maintenir ladite composition topique au contact de la peau d'un sujet, de préférence la peau d'un être humain.
